# EUROPEAN PATENT APPLICATION

(11) **EP 1 364 953 A1**
(43) Date of publication of application: **26.11.2003**
(21) Application number: 02711292.9
(22) Date of filing: 01.02.2002
(51) Int. Cl.: C07D 487/14, C07D 487/04, A61K 31/519, A61P 43/00, A61P 27/06, A61P 9/12, A61P 37/08, A61P 29/00, A61P 9/10, A61P 35/02, A61P 17/04, A61P 11/10, A61P 11/14, A61P 11/06

(54) **TRIAZOLOQUINAZOLINE AND PYRAZOLOTRIAZOLOPYRIMIDINE DERIVATIVES, MEDICINAL COMPOSITIONS, ADENOSINE A3 RECEPTOR AFFINITY AGENTS, OCULAR TENSION LOWERING AGENTS, PREPARATIONS FOR PREVENTING AND TREATING GLAUCOMA AND METHOD OF LOWERING OCULAR TENSION**

(30) Priority: 05.02.2001 JP 2001028831
(71) Applicant: OTSUKA PHARMACEUTICAL FACTORY, INC., Naruto-shi, Tokushima 772-8601 (JP)
(72) Inventor: OKAMURA, Takashi, Muya-cho, Naruto-shi, Tokushima 772-0017 (JP); KUROGI, Yasuhisa, Muya-cho, Naruto-shi, Tokushima 772-0017 (JP); NISHIKAWA, Hiroshi, Muya-cho, Naruto-shi, Tokushima 772-0017 (JP)
(74) Representative: HOFFMANN - EITLE
(86) International application number: JP0200874
(87) International publication number: WO02062801

(57) **Abstract**

The present invention provides triazoloquinazoline and pyrazolotriazolopyrimidine derivatives represented by the following general formula (1): 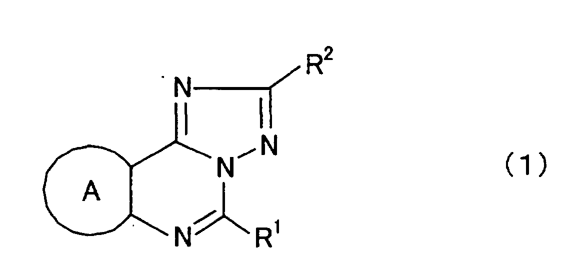 wherein R¹ and R² represent the same groups as those described in the description; and A represents a pyrazole or benzene ring which is optionally substituted with the groups described in the description. These derivatives exhibit an affinity to an adenosine A3 receptor and also have an intraocular-pressure reducing effect, and are useful for prevention or treatment of glaucoma.

## Description

### TECHNICAL HELD

The present invention relates to novel triazoloquinazoline and pyrazolotriazolopyrimidine derivatives which exhibit an affinity to an adenosine A3 receptor, a pharmaceutical composition containing the derivatives, an adenosine A3 receptor affinitive agent, an intraocular-pressure reducing agent, a pharmaceutical preparation for prevention and treatment of glaucoma, and an intraocular-pressure reducing method.

### BACKGROUD ART

J. Heterocyclic Chem., 31, 1171 (1994) discloses that 2-aryl-8-fluorobenzyl-1,2,4-triazolo[5,1-i]purine is useful as an adenosine A2 receptor antagonist.

Eur. J. Med. Chem., 28, 569 (1993) describes that a pyrazolotriazolopyrimidine derivative exhibits an adenosine A2 receptor antagonist effect. Also J. Med. Chem., 34(1), 281 (1991) describes that a triazoloquinazoline derivative exhibits high affinity to a benzodiazepine receptor.

An object of the present invention is to provide a novel compound having an affinity to an adenosine A3 receptor.

### DISCLOSURE OF THE INVENTION

To achieve the object described above, the present invention provides triazoloquinazoline and pyrazolotriazolopyrimidine derivatives represented by the following general formula (1): 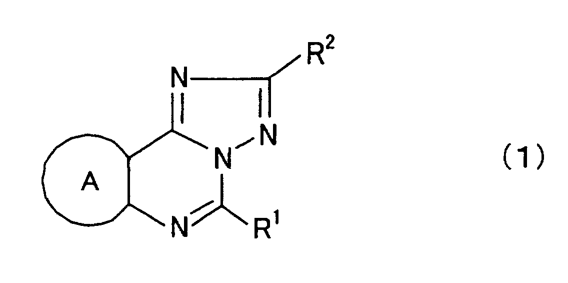 wherein R¹ represents a lower alkyl group, a phenyl group, a lower alkoxycarbonyl lower alkyl group, or a carboxy lower alkyl group; R² represents a pyridyl group, a furyl group, a thieriyl group, or a phenyl group which optionally has 1 to 3 groups selected from lower alkyl group, halogen atom, phenyl group, halogen-substituted lower alkyl group, hydroxy group, lower alkoxy group, N,N-di lower alkylamino group, lower alkylthio group, lower alkanoyloxy group and nitro group as a substituent; A represents a pyrazole ring which is optionally substituted with a group selected from lower alkyl group, phenyl lower alkyl group, lower alkoxycarbonyl lower alkyl group, carboxy lower alkyl group and hydroxy lower alkyl group as a substituent, or a benzene ring which is optionally substituted with 1 to 2 groups selected from halogen atom, lower alkyl group, nitro group and lower alkoxy group as a substituent; with the exception that A is a benzene ring, R² is a pyridyl group or a phenyl group, and R¹ is a methyl group, an ethyl group or a phenyl group.

These triazoloquinazoline and pyrazolotriazolopyrimidine derivatives of the present invention are novel compounds which have never been described in reference documents.

The pyrazolotriazolopyrimidine derivative in the present invention is represented by the following general formula (1-1): 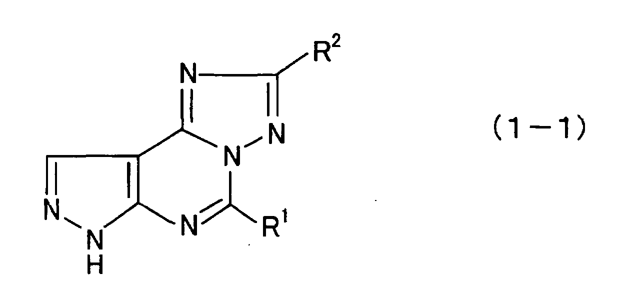 wherein R¹ and R² are as defined above.

In the present invention, it is particularly preferable that R² is a phenyl group which optionally have one group selected from lower alkyl group, halogen atom, halogen-substituted lower alkyl group and hydroxy group as a substituent, or phenyl group which has 1 to 3 lower alkoxy groups.

In that case, A is preferably a pyrazole ring, or a benzene ring which is optionally substituted with one halogen atom as a substituent. More preferably, R¹ is an n-butyl group and R² is a phenyl group which optionally has one group selected from lower alkoxy group, lower alkyl group, halogen atom and hydroxy group as a substituent.

Specifically, preferable compound is a compound selected from compound in which A is a pyrazole ring and R² is a phenyl group having any one of lower alkoxy group, lower alkyl group or halogen atom as a substituent, compound in which A is a benzene ring and R² is a phenyl group having one group selected from lower alkoxy group, halogen atom and hydroxy group as a substituent, and compound in which A is a benzene ring substituted with one halogen atom and R² is a phenyl group, and the compound includes the following compounds:
5-n-butyl-2-(4-chlorophenyl)pyrazolo[4,3-e]-1,2,4-triazolo[1,5-c]pyrimidine,
5-n-butyl-2-(4-chlbrophenyl)-1,2,4-triazolo[1,5-c]quinazoline,
5-n-butyl-2-(4-ethoxyphenyl)-1,2,4-triazolo[1,5-c]quinazoline, and
5-n-butyl-9-chloro-2-phenyl-1,2,4-triazolo[1,5-c]quinazoline.

The compound of the present invention is more preferably a compound wherein R² is a phenyl group which has a lower alkyl group or a halogen atom at the 4-position, and the compound include the following compounds:
5-n-butyl-2-(4-chlorophenyl)pyrazolo[4,3-e]-1,2,4-triazolo[1,5-c]pyrimidine,
5-n-butyl-2-(4-fluorophenyl)pyrazolo[4,3-e]-1,2,4-triazolo[1,5-c]pyrimidine,
5-n-butyl-2-(4-methylphenyl)pyrazolo[4,3-e]1,2,4-triazolo[1,5-c]pyrimidine,
5-n-butyl-2-(4-chlorophenyl)-1,2,4-triazolo[1,5-c]quinazoline,
5-n-butyl-2-(4-bromophenyl)-1,2,4-triazolo[1,5-c]quinazoline, and
5-n-butyl-2-(4-chlorophenyl)-9-chloro-1,2,4-triazolo[1,5-c]quinazoline.

The compound of the present invention is still more preferably 5-n-butyl-2-(4-methylphenyl)pyrazolo[4,3-e]-1,2,4-triazolo[1,5-c]pyrimidine or 5-n-butyl-2-(4-bromophenyl)-1,2,4-triazolo[1,5-c]quinazoline, among the compounds described above.

It is expected that the compound of the present invention is applied to antihypertensive agent, antiallergic agent, antiinflammatory agent, remedy for ischemic heart disease, remedy for leukemia, antipruritic, expectorant, cough remedy, remedy for asthma, analgesic, intraocular-pressure reducing agent and pharmaceutical preparation for prevention or treatment of glaucoma as a compound capable of binding with an adenosine A3 receptor because of its excellent affinity to an adenosine A3 receptor.

Therefore, the present invention provides a pharmaceutical composition comprising a compound selected from the triazoloquinazoline and pyrazolotriazolopyrimidine derivatives and a pharmaceutically acceptable carrier.

Also the present invention provides an adenosine A3 receptor affinitive agent comprising a compound selected from the triazoloquinazoline and pyrazolotriazolopyrimidine derivatives as an active ingredient.

Furthermore, the present invention provides an intraocular-pressure reducing method, which comprises administering an effective amount of a compound selected from the triazoloquinazoline and pyrazolotriazolopyrimidine derivatives to a patient having an enhanced intraocular pressure.

### BEST MODE FOR CARRYING OUT THE INVENTION

In the present invention, the lower alkyl group includes, for example, straight-chain or branched lower alkyl groups having 1 to 6 carbon atoms, such as methyl, ethyl, propyl, butyl, isobutyl, tert-butyl, pentyl, and hexyl.

The lower alkoxy group includes, for example, straight-chain or branched lower alkoxy groups having 1 to 6 carbon atoms, such as methoxy, ethoxy, propoxy, butoxy, tert-butoxy, pentyloxy, and hexyloxy.

The halogen atom includes fluorine, chlorine, bromine, or iodine. The pyridyl group includes 2-pyridyl, 3-pyridyl or 4-pyridyl.

The furyl group includes 2-furyl or 3-furyl.

The thienyl group includes 2-thienyl or 3-thienyl.

The alkoxycarbonyl lower alkyl group includes C₁-C₆ lower alkyl groups substituted with a C₁-C₆ lower alkoxycarbonyl group, such as methoxycarbonylmethyl, ethoxycarbonylmethyl, propoxycarbonylmethyl, butoxycarbonylmethyl, pentyloxycarbonylmethyl, hexyloxycarbonylmethyl, 2-methoxycarbonylethyl, 1-methoxycarbonylethyl, 3-methoxycarbonylpropyl, 4-methoxycarbonylbutyl, 5-methoxycarbonylpentyl, and 6-methoxycarbonylhexyl.

The carboxy lower alkyl group includes C₁-C₆ lower alkyl groups substituted with a carboxy group, such as carboxymethyl, 2-carboxyethyl, 1-carboxyethyl, 3-carboxypropyl, 4-carboxybutyl, 5-carboxypentyl, and 6-carboxyhexyl.

The hydroxy lower alkyl group includes C₁-C₆ lower alkyl groups substituted with a hydroxy group, such as hydroxymethyl, 2-hydroxyethyl, 1-hydroxyethyl, 3-hydroxypropyl, 4-hydroxybutyl, 5-hydroxypentyl, and 6-hydroxyhexyl.

The lower alkanoyloxy group includes lower alkanoyloxy groups wherein a C₁-C₆ straight-chain or branched lower alkyl group is bound to carbonyl carbon, such as acetoxy, propionyloxy, butyryloxy, isobutyryloxy, valeryloxy, pivaloyloxy, hexanoyloxy, and heptanoyloxy.

The phenyl which optionally has 1 to 3 groups selected from lower alkyl group, halogen atom, phenyl group, halogen-substituted lower alkyl group, hydroxy group, lower alkoxy group, N,N-di lower alkylamino group, lower alkylthio group, lower alkanoyloxy group and nitro group as a substituent includes, for example, phenyl groups which optionally have 1 to 3 substituents selected from C₁-C₆ alkyl group, halogen atom, phenyl group, halogen-substituted C₁-C₆ alkyl group, hydroxy group, C₁-C₆ alkoxy group, N,N-di C₁-C₆ alkylamino group, C₁-C₆ alkylthio group, C₂-C₇ alkanoyloxy group and nitro group as a substituent, such as 2-methylphenyl, 3-methylphenyl, 4-methylphenyl, 4-ethylphenyl, 4-propylphenyl, 4-isopropylphenyl, 4-butylphenyl, 4-t-butylphenyl, 4-pentylphenyl, 4-hexylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 3,4-diethylphenyl, 3,4-dipropylphenyl, 3,4-dibutylphenyl, 3,4-dipentylphenyl, 3,4-dihexylphenyl, 3,4,5-trimethylphenyl, 2,3,4-trimethylphenyl, 2,3,5-trimethylphenyl, 2,3,6-trimethylphenyl, 2,4,6-trimethylphenyl, 2,4,5-trimethylphenyl, 3,4,5-triethylphenyl, 3,4,5-tripropylphenyl, 3,4,5-tributylphenyl, 3,4,5-tripentylphenyl, 3,4,5-trihexylphenyl, 2-chlorophenyl, 3-chlorophenyl, 4-chlorophenyl, 2-bromophenyl, 3-bromophenyl, 4-bromophenyl, 2-iodophenyl, 3-iodophenyl, 4-iodophenyl, 2-fluorophenyl, 3-fluorophenyl, 4-fluorophenyl, 2,4-dichlorophenyl, 2,3-dichlorophenyl, 3,5-dichlorophenyl, 3,4-dichlorophenyl, 2,5-dichlorophenyl, 2,6-dichlorophenyl, 2,4-difluorophenyl, 2,4-dibromophenyl, 2,4-diiodophenyl, 3,4,5-trichlorophenyl, 2,4,6-trichlorophenyl, 4-biphenylyl, 3-biphenylyl, 2-biphenylyl, 4-trifluoromethylphenyl, 4-pentafluoroethylphenyl, 4-heptafluoropropylphenyl, 4-nonafluorobutylphenyl, 4-undecafluoropentylphenyl, 4-tridecafluorohexylphenyl, 3-trifluoromethylphenyl, 2-trifluoromethylphenyl, 2-hydroxyphenyl, 3-hydroxyphenyl, 4-hydroxyphenyl, 2,3-dihydroxyphenyl, 2,4-dihydroxyphenyl, 2,5-dihydroxyphenyl, 2,6-dihydroxyphenyl, 3,4-dihydroxyphenyl, 3,5-dihydroxyphenyl, 3,4,5-trihydroxyphenyl, 2,3,4-trihydroxyphenyl, 2,3,5-trihydroxyphenyl, 2,3,6-trihydroxyphenyl, 2,4,6-trihydroxyphenyl, 2,4,5-trihydroxyphenyl, 2-methoxyphenyl, 3-methoxyphenyl, 4-methoxyphenyl, 4-ethoxyphenyl, 4-propoxyphenyl, 4-butoxyphenyl, 4-pentyloxyphenyl, 4-hexyloxyphenyl, 2,3-dimethoxyphenyl, 2,4-dimethoxyphenyl, 2,5-dimethoxyphenyl, 2,6-dimethoxyphenyl, 3,4-dimethoxyphenyl, 3,5-dimethoxyphenyl, 3,4-diethoxyphenyl, 3,4-dipropoxyphenyl, 3,4-dibutoxyphenyl, 3,4-dipentyloxyphenyl, 3,4-dihexyloxyphenyl, 3,4,5-trimethoxyphenyl, 2,3,4-trimethoxyphenyl, 2,3,5-trimethoxyphenyl, 2,3,6-trimethoxyphenyl, 2,4,6-trimethoxyphenyl, 2,4,5-trimethoxyphenyl, 3,4,5-triethoxyphenyl, 3,4,5-tripropoxyphenyl, 3,4,5-tributoxyphenyl, 3,4,5-tripentyloxyphenyl, 3,4,5-trihexyloxyphenyl, 4-(N,N-dimethylamino)phenyl, 4-(N,N-diethylamino)phenyl, 4-(N,N-dipropylamino)phenyl, 4-(N,N-dibutylamino)phenyl, 4-(N,N-dipentylamino)phenyl, 4-(N,N-dihexylamino)phenyl, 3-(N,N-dimethylamino)phenyl, 2-(N,N-dimethylamino)phenyl, 4-methylthiophenyl, 4-ethylthiophenyl, 4-propylthiophenyl, 4-butylthiophenyl, 4-pentylthiophenyl, 4-hexylthiophenyl, 3-methylthiophenyl, 2-methylthiophenyl, 2-nitrophenyl, 3-nitrophenyl, 4-nitrophenyl, 2,3-dinitrophenyl, 2,4-dinitrophenyl, 2,5-dinitrophenyl, 2,6-dinitrophenyl, 3,4-dinitrophenyl, 3,5-dinitrophenyl, 3,4,5-trinitrophenyl, 2,3,4-trinitrophenyl, 2,3,5-trinitrophenyl, 2,3,6-trinitrophenyl, 2,4,6-trinitrophenyl, 2,4,5-trinitrophenyl, 4-methoxy-3-methylphenyl, 4-methoxy-2-methylphenyl, 3-methoxy-2-methylphenyl, 4-methoxy-3,5-dimethylphenyl, 4-hydroxy-3-methylphenyl, 4-hydroxy-2-methylphenyl, 3-hydroxy-2-methylphenyl, 2-hydroxy-4-methylphenyl, 2-hydroxy-4-methoxyphenyl, 4-hydroxy-3,5-dimethylphenyl, 3,5-di-t-butyl-4-hydroxyphenyl, 4-hydroxy-3,5-dimethoxyphenyl, 3,5-dihydroxy-4-methoxyphenyl, 4-chloro-3-methoxyphenyl, 3-chloro-4-methoxyphenyl, 4-chloro-2-hydroxyphenyl, 4-chloro-3-hydroxyphenyl, 4-chloro-3-methylphenyl, 3-chloro-4-methylphenyl, 4-chloro-3,5-dimethoxyphenyl, 4-chloro-3,5-dimethylphenyl, 4-acetoxyphenyl, 4-propionyloxyphenyl, 4-butyryloxyphenyl, 4-isobutyryloxyphenyl, 4-valeryloxyphenyl, 4-pivaloyloxyphenyl, 4-hexanoyloxyphenyl, 4-heptanoyloxyphenyl, 3,5-diacetoxyphenyl, 3,4-diacetoxyphenyl, 3,4,5-triacetoxyphenyl, and 2,4,6-triacetoxyphenyl, in addition to the phenyl group.

In case the benzene ring itself is represented as a phenylene group, the benzene ring which is optionally substituted with 1 to 2 groups selected from halogen atom, lower alkyl group, nitro group and lower alkoxy group as a substituent includes, for example, benzene rings which are optionally substituted with 1 to 2 groups selected from halogen atom, C₁-C₆ alkyl group, nitro group and C₁-C₆ alkoxy group as a substituent, such as 2-chloro-1,6-phenylene, 3-chloro-1,6-phenylene, 4-chloro-1,6-phenylene, 2-bromo-1,6-phenylene, 3-bromo-1,6-phenylene, 2-iodo-1,6-phenylene, 3-iodo-1,6-phenylene, 2-fluoro-1,6-phenylene, 3-fluoro-1,6-phenylene, 4-fluoro-1,6-phenylene, 2,4-dichloro-1,6-phenylene, 2,3-dichloro-1,6-phenylene, 3,4-dichloro-1,6-phenylene, 2,5-dichloro-1,6-phenylene, 2,4-difluoro-1,6-phenylene, 2,4-dibromo-1,6-phenylene, 2,4-diiodo-1,6-phenylene, 2-methyl-1,6-phenylene, 3-methyl-1,6-phenylene, 4-methyl-1,6-phenylene, 2-ethyl-1,6-phenylene, 2-propyl-1,6-phenylene, 2-isopropyl-1,6-phenylene, 2-butyl-1,6-phenylene, 2-t-butyl-1,6-phenylene, 2-pentyl-1,6-phenylene, 2-hexyl-1,6-phenylene, 2,3-dimethyl-1,6-phenylene, 2,4-dimethyl-1,6-phenylene, 2,5-dimethyl-1,6-phenylene, 3,4-dimethyl-1,6-phenylene, 3,4-diethyl-1,6-phenylene, 3,4-dipropyl-1,6-phenylene, 3,4-dibutyl-1,6-phenylene, 3,4-dipentyl-1,6-phenylene, 3,4-dihexyl-1,6-phenylene, 2-methoxy-1,6-phenylene, 3-methoxy-1,6-phenylene, 4-methoxy-1,6-phenylene, 3-ethoxy-1,6-phenylene, 3-propoxy-1,6-phenylene, 3-butoxy-1,6-phenylene, 3-pentyl-1,6-phenylene, 3-hexyl-1,6-phenylene, 2,3-dimethoxy-1,6-phenylene, 2,4-dimethoxy-1,6-phenylene, 2,5-dimethoxy-1,6-phenylene, 3,4-dimethoxy-1,6-phenylene, 3,4-diethoxy-1,6-phenylene, 3,4-dipropoxy-1,6-phenylene, 3,4-dibutoxy-1,6-phenylene, 3,4-dipentyloxy-1,6-phenylene, 3,4-dihexyloxy-1,6-phenylene, 4-methoxy-3-methyl-1,6-phenylene, 4-methoxy-2-methyl-1,6-phenylene, 3-methoxy-2-methyl-1,6-phenylene, 4-chloro-3-methyl-1,6-phenylene, 4-chloro-2-methyl-1,6-phenylene, 3-chloro-2-methyl-1,6-phenylene, 2-chloro-4-methyl-1,6-phenylene, 2-chloro-4-methoxy-1,6-phenylene, 4-chloro-3-methyl-1,6-phenylene, 4-chloro-3-di-t-butyl-1,6-phenylene, 4-chloro-3-methoxy-1,6-phenylene, 3-chloro-4-methoxy-1,6-phenylene, 4-chloro-3-methoxy-1,6-phenylene, 4-butoxy-3-chloro-1,6-phenylene, 2-chloro-5-methoxy-1,6-phenylene, 2-nitro-1,6-phenylene 3-nitro-1,6-phenylene, 4-nitro-1,6-phenylene, and 5-nitro-1,6-phenylene, in addition to the 1,6-phenylene group.

The pyrazole ring which is optionally substituted with a group selected from lower alkyl group, phenyl lower alkyl group, lower alkoxycarbonyl lower alkyl group, carboxy lower alkyl group and hydroxy lower alkyl group as a substituent include, for example, pyrazole rings wherein hydrogen bound to either of nitrogen atom is substituted with the respective groups described above, in addition to the non-substituted pyrazole ring.

The compound (1a) of the present invention can be prepared by the following reaction scheme-1. 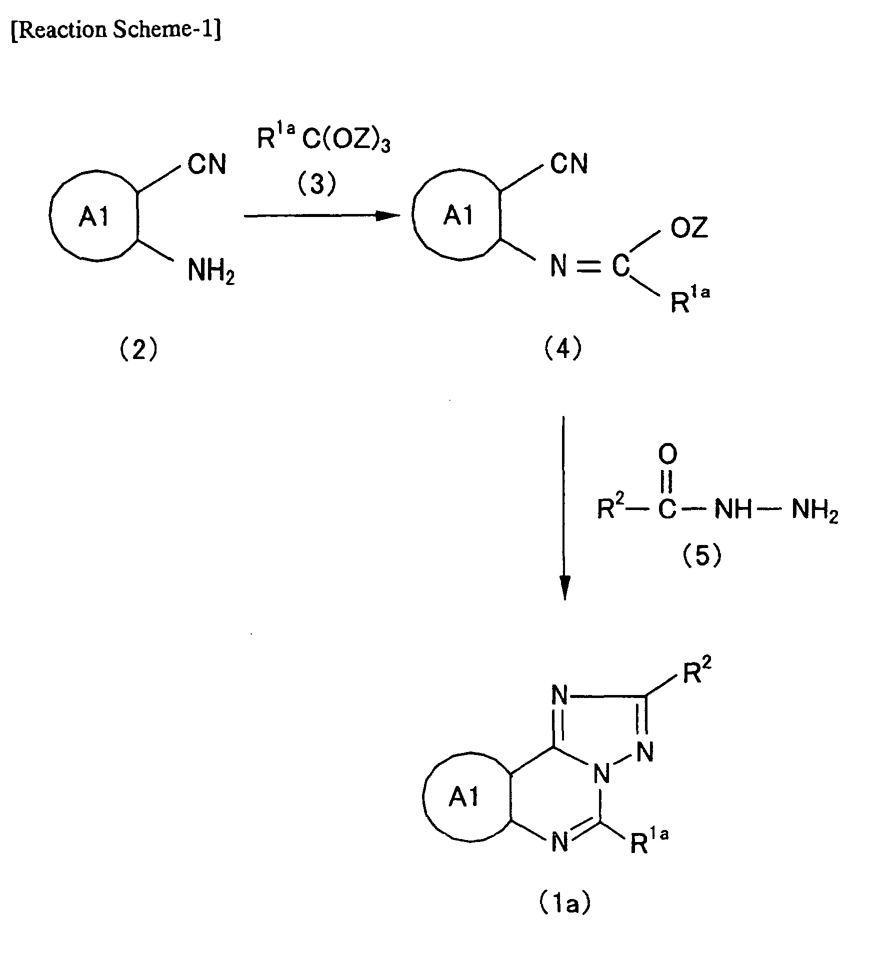 wherein R² is as defined above; R^{1a} represents a lower alkyl group or a phenyl group; A1 represents a non-substituted pyrazole ring, or a benzene ring which is optionally substituted with 1 to 2 groups selected from halogen atom, lower alkyl group, nitro group and lower alkoxy group as a substituent; and Z represents a lower alkyl group.

First, a compound represented by the formula (2) is reacted with an orthoester derivative represented by the formula (3) to obtain an imino ester represented by the formula (4). This reaction is carried out by adding the orthoester derivative (3) in an equimolar amount or more relative to the amount of the compound (2) and heating at a temperature within a range from room temperature to reflux temperature for about 10 minutes to 24 hours in the absence of a solvent, or in an inert solvent. As the inert solvent, for example, N,N-dimethylformamide (DMF), N,N-dimethylacetamide (DMA), dimethyl sulfoxide (DMSO), methanol, diphenyl ether, xylene, and diethylene glycol dimethyl ether can be used.

The resulting imino ester derivative (4) is reacted with an acyl hydrazine derivative (5), after the imino ester derivative is purified according to a conventional method or not, to obtain the compounds (1a) of the present invention.

This reaction is carried out by adding the acyl hydrazine derivative (5) in an equimolar amount or more relative to the amount of the imino ester derivative (4) in an inert solvent and heating at a temperature within a range from 50°C to reflux temperature for about 1 to 50 hours. The inert solvent includes the same solvents as those described above.

The above-described two-stage reaction can also be carried out simultaneously in the same reaction vessel. For example, the reaction can be carried out by adding the acyl hydrazine derivative (5) in the reaction mixture of the compound (2) and the orthoester derivative (3) and heating in the same manner. In this case, a trace amount of an acid catalyst such as p-toluenesulfonic acid, camphorsulfonic acid, sulfuric acid or trifluoroacetic acid is preferably added in the reaction system.

The compound (1a') of the present invention is converted into the compound (1b of the present invention by the hydrolysis reaction, the reaction with an acid chloride (7) or the cyclization reaction, as shown in the following reaction scheme-2. 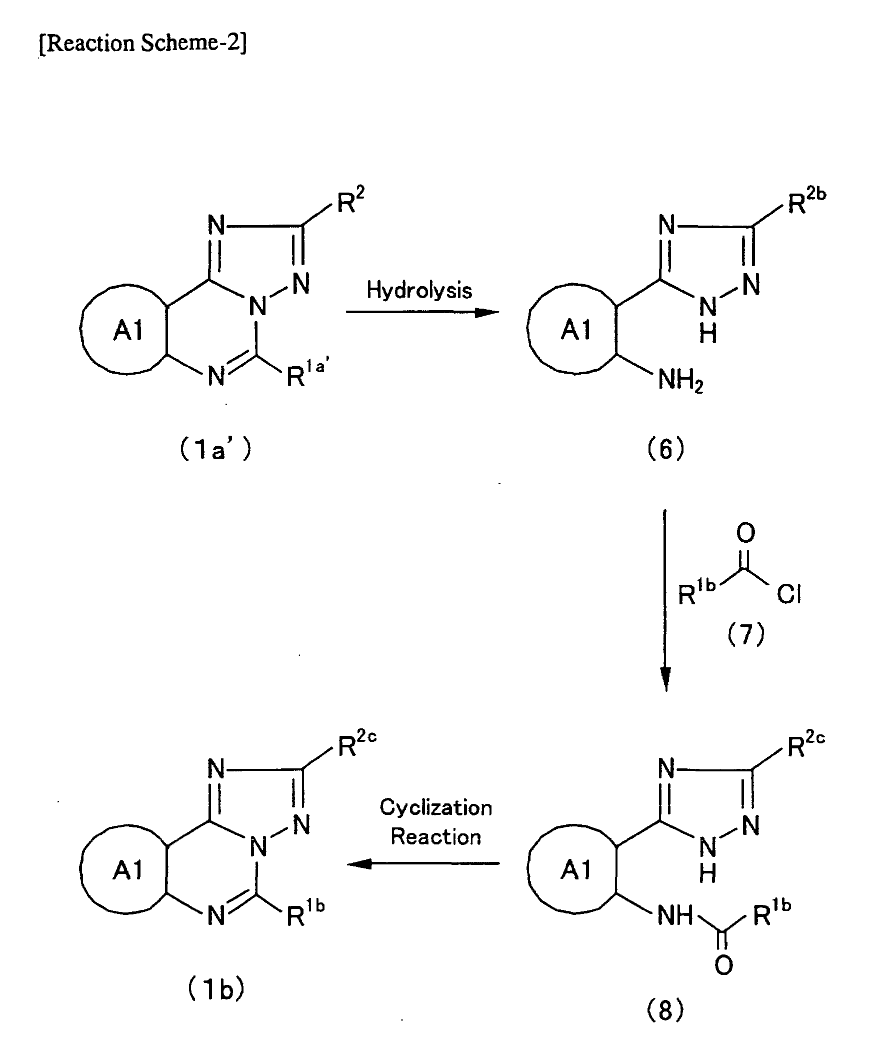 wherein R² and A1 are as defined above; R^{1a'} represents a lower alkyl group; R^{1b} represents a lower alkyl group, a phenyl group or a lower alkoxycarbonyl lower alkyl group, R^{2b} represents a pyridyl group, a furyl group, a thienyl group, or a phenyl group which optionally have 1 to 3 groups selected from lower alkyl group, halogen atom, phenyl group, halogen-substituted lower alkyl group, hydroxy group, lower alkoxy group, N,N-di lower alkylamino group, lower alkylthio group and nitro group as a substituent; and R^{2c} represents a pyridyl group, a furyl group, a thienyl group, or a phenyl group which optionally have 1 to 3 groups selected from lower alkyl group, halogen atom, phenyl group, halogen-substituted lower alkyl group, lower alkoxy group, N,N-di lower alkylamino group, lower alkylthio group, lower alkanoyloxy group and nitro group as a substituent.

First, the compound (1a') is converted into an amine compound (6) by refluxing in an aqueous solution of mineral acid such as hydrochloric acid or sulfuric acid for 5 minutes to 50 hours.

Then, the amine compound (6) is acylated. This acylation can be carried out by reacting the amine compound (6) with the acid chloride (7) in an amine-based inert solvent such as pyridine, lutidine, triethylamine, or 4-(N,N-dimethylamino)pyridine. In this reaction, the acid chloride (7) is used in an equimolar amount or more and the reaction is completed within about 10 minutes to 3 hours at a temperature within a range from 0°C to reflux temperature. Since a compound substituted with a plurality of acyl groups may be included sometime in the acylation reaction, the inclusion can optionally be converted into a desired monoacyl compound (8) by refluxing the product, together with a catalytic amount of an alkaline such as anhydrous potassium carbonate or anhydrous sodium carbonate, in an inert solvent such as methanol or ethanol for about 10 minutes to 2 hours.

Subsequently, the monoacyl compound (8) thus obtained is converted into a compound (1b) of the present invention by the cyclization reaction. The cyclization reaction is carried out by reacting the monoacyl compound (8) with a halogenated trialkylsilane in an inert solvent in the presence of a base.

As the inert solvent, for example, there can be used aromatic and aliphatic hydrocarbons such as benzene, toluene, xylene, and petroleum ether; ethers such as diethyl ether and tetrahydrofuran; halogenated hydrocarbons such as dichloromethane, chloroform, carbon tetrachloride, and 1,2-dichloroethane; and aliphatic nitriles such as acetonitrile. As the base, for example, tertiary amine such as triethylamine, diisopropylamine, N,N-diethylaniline, N-methyl morpholine, pyridine, or 4-(N,N-dimethylamino)pyridine can be preferably used. As the halogenated trialkylsilane, for example, chlorotrialkylsilane such as chlorotrimethylsilane, chlorotriethylsilane, chloroethyldimethylsilane, chlorodimethylpropylsilane, chlorobutyldimethylsilane, chlorotripropylsilane, tributylchlorosilane, or chloroethylmethylpropylsilane can be preferably used.

The amount of the halogenated trialkylsilane and base to be used is not specifically limited, but is generally controlled to an equal equivalent weight or more, and preferably from 3- to 20-fold equivalent weight relative to the amount of the monoacyl compound (8). The cyclization reaction is usually completed within about 0.5 to 100 hours at a temperature within a range from 0 to 100°C.

Among the starting materials (1a') in the reaction scheme-2, the cyclization reaction simultaneously proceeds when using the acid chloride (7) in the amount of 3-fold equivalent weight relative to the amount of a compound wherein A1 is a benzene ring which is optionally substituted with 1 to 2 groups selected from halogen atom, lower alkyl group, nitro group and lower alkoxy group as a substituent in the acylation reaction after hydrolysis. Therefore, it becomes unnecessary to carry out the cyclization reaction in that case.

The compound (1c) of the present invention can be converted into compounds (1d-1) and (1d-2) wherein a substituent is introduced into the pyrazole ring by treating with a halide (9), as shown in the following reaction scheme-3. 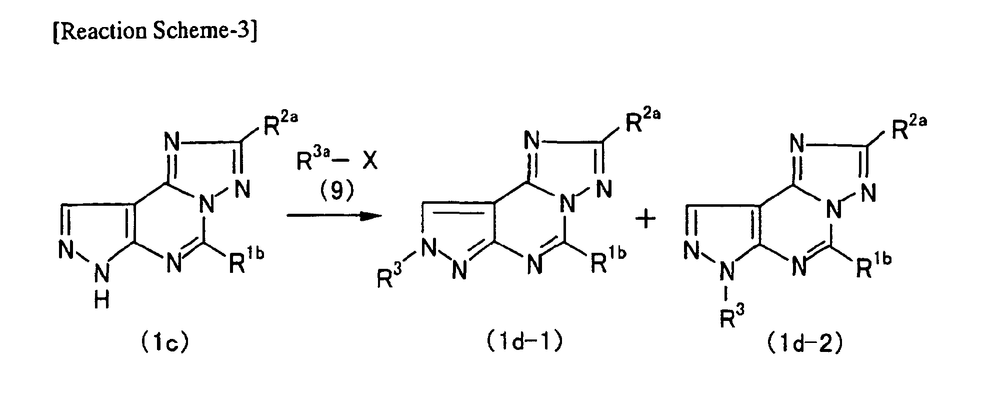 wherein R^{1b} is as defined above; R^{2a} represents a pyridyl group, a furyl group, a thienyl group, or a phenyl group which optionally has 1 to 3 groups selected from lower alkyl group, halogen atom, phenyl group, halogen-substituted lower alkyl group, lower alkoxy group, N,N-di lower alkylamino group, lower alkylthio group, lower alkanoyloxy group and nitro group as a substituent; R³ represents a lower alkyl group, a phenyl lower alkyl group, a lower alkoxycarbonyl lower alkyl group, or a hydroxy lower alkyl group, R^{3a} represents a lower alkyl group, a phenyl lower alkyl group, a lower alkoxycarbonyl lower alkyl group, or a trimethylsilyloxy-lower alkyl group; and X represents a halogen atom.

The conversion is carried out by reacting with an equal equivalent weight or more of a halide (9) in an inert solvent such as DMF, DMA, or DMSO in the presence of an equal equivalent weight or more of an alkali such as anhydrous potassium carbonate or anhydrous sodium carbonate at a temperature within a range from 0°C to room temperature for 2 to 50 hours.

When using, as the halide (9), a compound wherein R^{3a} is a trimethylsilyloxy-lower alkyl group, contact of the compound with water in a post treatment causes elimination of the trimethylsilyl group, and thus the trimethylsilyloxy-lower alkyl group is converted into the corresponding hydroxy lower alkyl group.

The compound (1e) of the present invention can be converted into a compound (1f) by hydrolysis, as shown in the reacton scheme-4. 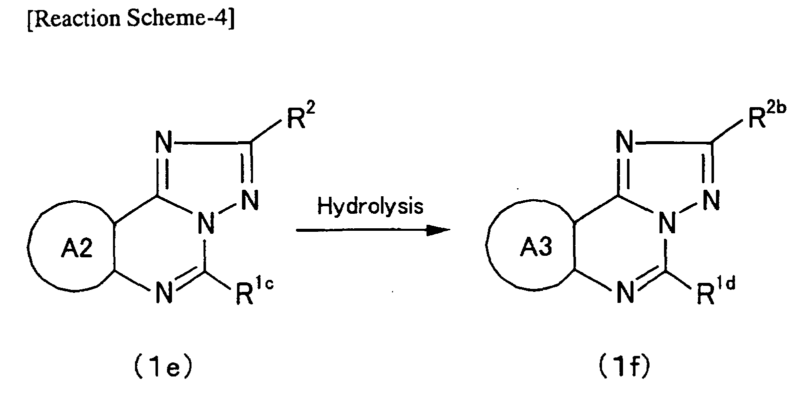 wherein R² and R^{2b} are as defined above; R^{1c} represents a lower alkyl group, a phenyl group, or a lower alkoxycarbonyl lower alkyl group; R^{1d} represents a lower alkyl group, a phenyl group, or a carboxy lower alkyl group; A2 represents a pyrazole ring which is optionally substituted with a group selected from lower alkyl group, phenyl lower alkyl group, lower alkoxycarbonyl lower alkyl group and hydroxy lower alkyl group as a substituent, or a benzene ring which is optionally substituted with 1 to 2 groups selected from halogen atom, lower alkyl group, nitro group and lower alkoxy group as a substituent; and A3 represents a pyrazole ring which is optionally substituted with a group selected from lower alkyl group, phenyl lower alkyl group, carboxy lower alkyl group and hydroxy lower alkyl group as a substituent, or a benzene ring which which is optionally substituted with 1 to 2 groups selected from halogen atom, lower alkyl group, nitro group and lower alkoxy group as a substituent; provided that the substituents satisfy at least one of (i) to (iii):
(i) R^{1c} is a lower alkoxycarbonyl lower alkyl group,
(ii) A2 is a pyrazole ring substituted with a lower alkoxycarbonyl lower alkyl group, and
(iii) R² is a phenyl group having a lower alkanoyloxy group.

The lower alkoxycarbonyl lower alkyl group and/or the lower alkanoyloxy group contained in the compound (1e) of the present invention are hydrolyzed by the hydrolysis reaction to form the corresponding carboxy lower alkyl group and/or hydroxy group, thereby obtaining a compound (1f).

The reaction is carried out by treating with a solution of an alkaline such as sodium hydroxide or potassium hydroxide in an inert solvent such as methanol or ethanol. The amount of the alkaline is preferably controlled to an equal equivalent weight or more and the reaction is usually completed within about 0.5 to 10 hours at a temperature within a range from about 0°C to room temperature.

The desired object in each process of the above reaction schemes 1 to 4 can be easily isolated and purified by a conventional separation means. The separation means includes adsorption chromatography, preparative thin-layer chromatography, recrystallization, solvent extraction or the like. When A is a pyrazole ring in the compounds (1) of the present invention prepared as described above, it is considered that the compound includes the following four structural formulas as a tautomer and the tautomer can be represented by any of the structural formulas. 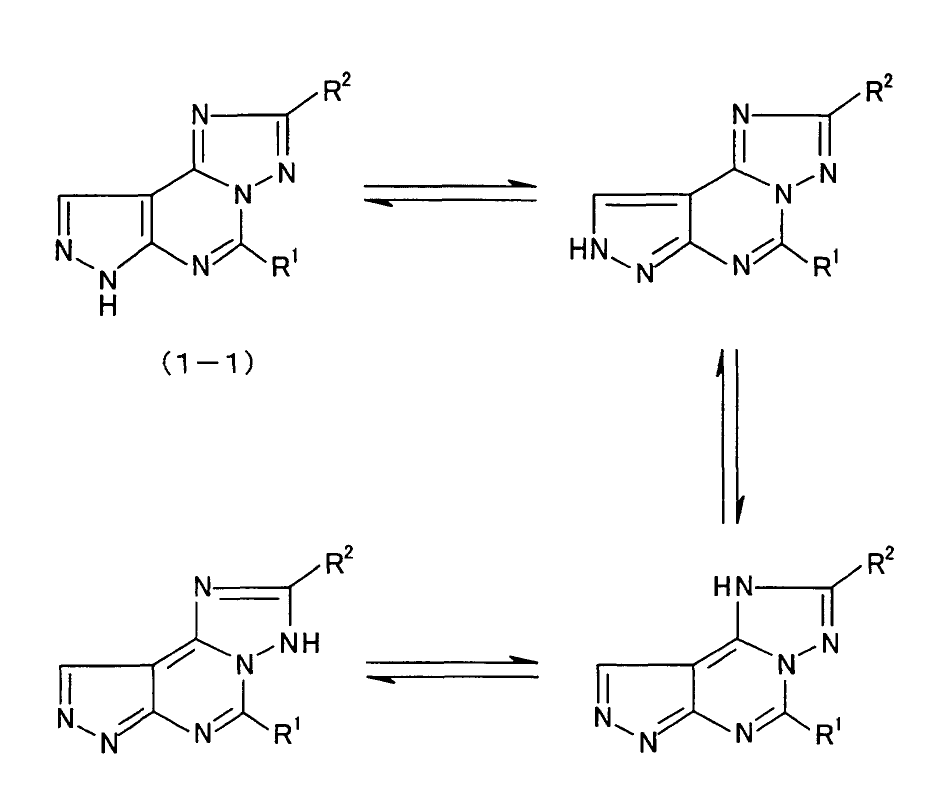 wherein R¹ and R² are as defined above.

The compounds (1) of the present invention can be formed into pharmaceutically acceptable acid addition salts, and these salts are also included in the present invention. The acid capable of forming these acid salts includes, for example, inorganic acids such as hydrochloric acid, hydrobromic acid, and sulfuric acid; and organic acids such as oxalic acid, fumaric acid, maleic acid, tartaric acid, citric acid, and p-toluenesulfonic acid. The acid addition salts can be formed by a conventional method.

Among the compounds of the present invention, the pyrazolotriazolopyrimidine derivative can be formed into alkaline metal salts such as sodium salt and potassium salt; alkaline earth metal salts such as calcium salt and magnesium salt; and copper salts by a conventional method, and these salts can also be included in the present invention.

The compounds (1) of the present invention are used in the form of a general pharmaceutical preparation by using, together with a suitable non-toxic preparation carrier. The preparation carrier include diluents and excipients, such as fillers, extenders, binders, humectants, disintegrators, surfactants, and lubricants, which are usually used according to the service form of the preparation, and these are appropriately selected and used according to the unit dosage form of the resulting preparation.

As the unit dosage form of the pharmaceutical preparation using the compound (1) of the present invention, various forms can be selected according to the therapeutic purposes and typical examples thereof include tablets, pills, powders, liquid preparations, suspensions, emulsions, granules, capsules, suppositories, injections (e.g. liquid preparations, suspensions, etc.), ointments, and opthalmic solutions.

In case of forming into the form of tablets, there can be used, as the preparation carrier, excipients such as lactose, sucrose, sodium chloride, glucose, urea, starch, calcium carbonate, kaolin, crystalline cellulose, silicic acid, and potassium phosphate; binders such as water, ethanol, propanol, simple syrup, glucose solution, starch solution, gelatin solution, carboxymethylcellulose, hydroxypropylcellulose, methylcellulose, and polyvinyl pyrrolidone; disintegrators such as sodium carboxymethylcellulose, calcium carboxymethylcellulose, low substituted hydroxypropylcellulose, dried starch, sodium alginate, agar powder, laminaran powder, sodium hydrogencarbonate, and calcium carbonate; surfactants such as polyoxyethylene sorbitan fatty acid esters, sodium lauryl sulfate, and monoglyceride stearate; disintegration inhibitors such as sucrose, stearin, cacao butter, and hydrogenated oil; absorption accelerators such as quaternary ammonium base and sodium lauryl sulfate; humectants such as glycerin and starch; adsorbents such as starch, lactose, kaolin, bentonite, and colloidal silicic acid; and lubricants such as purified talc, stearate, powdered boric acid, and polyethylene glycol.

If necessary, tablets can be formed into tablets coated with a common coating, for example, sugar-coated tablets, gelatin-coated tablets, enteric coated tablets, film coating tablets, double layered tablets, or mutilayer tablets.

In case of forming into the form of pills, there can be used, as the preparation carrier, excipients such as glucose, lactose, starch, cacao butter, hardened vegetable oil, kaolin, and talc; binders such as gum arabic, powdered tragacanth, gelatin, and ethanol; and disintegrators such as laminaran and agar.

In case of forming into the form of suppositories, there can be used, as the preparation carrier, polyethylene glycol, cacao butter, higher alcohol, esters of higher alcohol, gelatin, and semi-synthesized glyceride.

Capsules are usually prepared by mixing the compound (1) of the present invention with various pharmaceutical preparations and filling a hard gelatin capsule or a soft gelatin capsule with the mixture.

In case of preparing as injections such as liquid preparations, emulsions or suspension, these are preferably sterilized and are isotonic with blood. In case of forming into the form of injections, there can be used, as the diluent, water, ethyl alcohol, macrogol, propylene glycol, ethoxylated isostearyl alcohol, polyoxylated isostearyl alcohol, or polyoxyethylene sorbitan fatty acid esters. In this case, salt, glucose or glycerin may be contained in an enough amount to prepare an isotonic solution and common solubilizers, buffer agents or soothing agents may also be added.

If necessary, the pharmaceutical preparation further contains colorants, preservatives, perfumes, flavors, sweeteners, or other drugs.

In case of forming into the form of ointments such as paste, cream, or gel, there can be used, as the diluent, white soft paraffin, paraffin, glycerin, cellulose derivative, polyethylene glycol, silicon, and bentonite.

The amount of the compound (1) of the present invention to be incorporated in the pharmaceutical preparation is not specifically limited and appropriately selected from a wide range, but is preferably within a range from about 1 to 85% by weight based on the pharmaceutical preparation.

Eye drops are usually prepared by a conventional method using sterilized distilled water as a base; buffer agents such as sodium dihydrogenphosphate and sodium monohydrogenphosphate; isotonizing agents such as sodium chloride and concentrated glycerin; buffering agents such as sodium phosphate and sodium acetate; surfactants such as polyoxyethylene sorbitan monooleatae, polyoxy 40 stearate and polyocyethylene hardened castor oil; solubilizers such as sodium carboxymethylcellulose, polyoxyethylene lauryl ether, polyoxyethylene oleyl ether, polyethylene glycol monolaurate, and polyethylene glycol monooleate; stabilizers such as sodium citrate and sodium edetate; and antiseptics such as benzatonium chloride, chlorobutanol, benzalkonium chloride, and paraben. The pH is not specifically limited as far as it is within an ophthalmologically acceptable range, and is preferably within a range from 4 to 8.

The administration method of the pharmaceutical preparation is not specifically limited, but is appropriately decided according to the form of preparations, age of patients, sex and other conditions, or conditions of diseases. For example, tablets, pills, liquid preparations, suspensions, granules and capsules are orally administered, while injections are intravenously administered alone or in combination with a conventional replenisher such as glucose or amino acid, or intramascularly, intracutaneously, subctaneously or intraperitoneally administered alone, if necessary. Furthermore, suppositories are intrarectally administered.

The dose of the pharmaceutical preparation varies depending on the administration method, age of patients, sex and other conditions, or conditions of diseases, but a dairy dose of the compound (1) of the present invention is usually within a range from about 0.5 to 20 mg/kg, and preferably from about 1 to 10 mg/kg. The pharmaceutical preparation can be administered 1 to 4 times per day.

### INDUSTRIAL APPLICABILITY

It is expected that the compound of the present invention is applied to antihypertensive agent, antiallergic agent, antiinflammatory agent, remedy for ischemic heart disease, remedy for leukemia, antipruritic, expectorant, cough remedy, remedy for asthma, analgesic, intraocular-pressure reducing agent and pharmaceutical preparation for prevention or treatment of glaucoma as a compound capable of binding with an adenosine A3 receptor because of its excellent affinity to an adenosine A3 receptor.

### EXAMPLES

The following Examples further illustrate the compounds of the present invention in detail.

### <Example 1>

### Preparation of 5-methyl-2-phenylpyrazolo[4,3-e]-1,2,4-triazolo[1,5-c]pyrimidine

0.5 g of 3-amino-4-cyanopyrazole was suspended in 3 mL of DMF and 0.61 g of trimethyl orthoacetate and 1 mg of p-toluenesulfonic acid (monohydrate), followed by stirring at 80°C for 45 minutes. To the reaction solution, 0.69 g of N-benzoyl hydrazine was further added, and then the mixture was refluxed for 14 hours. After the completion of the reaction, 10 mL of water was added when the reaction solution was cooled to about 100°C, followed by cooling to room temperature. The deposited crystal was collected by filtration and then washed with hydrous ethanol to obtain 0.91 g of a desired compound as a crystal.
Melting point: > 280°C

### <Examples 2 to 86>

In the same manner as in Example 1, the following compounds were prepared.
(Example 2) 5-ethyl-2-phenylpyrazolo[4,3-e]-1,2,4-triazolo[1,5-c]pyrimidine
   Melting point: 274 - 275.5°C
(Example 3) 2-phenyl-5-propylpyrazolo[4,3-e]-1,2,4-triazolo[1,5-c]pyrimidine
   Melting point: 247 to 248°C
(Example 4) 5-n-butyl-2-phenylpyrazolo[4,3-e]-1,2,4-triazolo[1,5-c]pyrimidine
   Melting point: 214 - 215°C
(Example 5) 5-n-butyl-2-(4-fluorophenyl)pyrazolo[4,3-e]-1,2,4-triazolo[1,5-c]pyrimidine
   Melting point: 228 to 230°C
(Example 6) 5-n-butyl-2-(4-chlorophenyl)pyrazolo[4,3-e]-1,2,4-triazolo[1,5-c]pyrimidine
   Melting point: 231 - 233°C
(Example 7) 2-(4-bromophenyl)-5-n-butylpyrazolo[4,3-e]-1,2,4-triazolo[1,5-c]pyrimidine
   Melting point: 257 - 258°C
(Example 8) 5-n-butyl-2-(4-methylphenyl)pyrazolo[4,3-e]-1,2,4-triazolo[1,5-c]pyrimidine
   Melting point: 242 to 243°C
(Example 9) 5-n-butyl-2-(4-t-butylphenyl)pyrazolo[4,3-e]-1,2,4-triazolo[1,5-c]pyrimidine
   Melting point: 197 - 199°C
(Example 10) 5-n-butyl-2-(4-trifluoromethylphenyl)pyrazolo[4,3-e]-1,2,4-triazolo[1,5-c]pyrimidine
   Melting point: 234 - 235°C
(Example 11) 2-(4-biphenylyl)-5-n-butylpyrazolo[4,3-e]-1,2,4-triazolo[1,5-c]pyrimidine
   Melting point: 249 - 250°C
(Example 12) 5-n-butyl-2-(4-hydroxyphenyl)pyrazolo[4,3-e]-1,2,4-triazolo[1,5-c]pyrimidine
   Melting point: > 280°C
(Example 13) 5-n-butyl-2-(4-ethoxyphenyl)pyrazolo[4,3-e]-1,2,4-triazolo[1,5-c]pyrimidine
   Melting point: 204 - 205.5°C
(Example 14) 5-n-butyl-2-(4-propoxyphenyl)pyrazolo[4,3-e]-1,2,4-triazolo[1,5-c]pyrimidine
   Melting point: 191 - 192°C
(Example 15) 5-n-butyl-2-(3,4,5-trimethoxyphenyl)pyrazolo[4,3-e]-1,2,4-triazolo[1,5-c]pyrimidine
   Melting point: 259 - 262°C
(Example 16) 5-n-butyl-2-[4-(N,N-dinnethylamino)phenyl]pyrazolo[4,3-e]-1,2,4-triazolo[1,5-c]pyrimidine
   Melting point: > 240°C (with decomposition)
(Example 17) 5-n-butyl-2-(4-nitrophenyl)pyrazolo[4,3-e]-1,2,4-triazolo[1,5-c]pyrimidine
   Melting point: 279 - 280.5°C
(Example 18) 5-n-butyl-2-(3-pyridyl)pyrazolo[4,3-e]-1,2,4-triazolo[1,5-c]pyrimidine
   Melting point: 217 - 219°C
(Example 19) 5-n-butyl-2-(2-furyl)pyrazolo[4,3-e]-1,2,4-triazolo[1,5-c]pyrimidine
   Melting point: 253 - 254.5°C
(Example 20) 5-n-butyl-2-(2-thienyl)pyrazolo[4,3-e]-1,2,4-triazolo[1,5-c]pyrimidine
   Melting point: 214 - 216°C
(Example 21) 2-phenyl-5-propyl-1,2,4-triazolo[1,5-c]quinazoline
   Melting point: 143 - 144°C
(Example 22) 5-n-butyl-2-phenyl-1,2,4-triazolo[1,5-c]quinazoline
   Melting point: 133 - 135°C
(Example 23) 5-n-butyl-2-(4-fluorophenyl)-1,2,4-triazolo[1,5-c]quinazoline
   Melting point: 167 - 169°C
(Example 24) 5-n-butyl-2-(2-chlorophenyl)-1,2,4-triazolo[1,5-c]quinazoline
   Melting point: 1045 - 105.5°C
(Example 25) 5-n-butyl-2-(3-chlorophenyl)-1,2,4-triazolo[1,5-c]quinazoline
   Melting point: 129 - 131°C
(Example 26) 5-n-butyl-2-(4-chlorophenyl)-1,2,4-triazolo[1,5-c]quinazoline
   Melting point: 160 - 161°C
(Example 27) 2-(4-bromophenyl)-5-n-butyl-1,2,4-triazolo[1,5-c]quinazoline
   Melting point: 169-171°C
(Example 28) 5-n-butyl-2-(4-methylphenyl)-1,2,4-triazolo[1,5-c]quinazoline
   Melting point: 153 - 154°C
(Example 29) 5-n-butyl-2-(4-t-butylphenyl)-1,2,4-triazolo[1,5-c]quinazoline
   Melting point: 106 - 108°C
(Example 30) 5-n-butyl-2-(4-trifluoromethylphenyl)-1,2,4-triazolo[1,5-c]quinazoline
   Melting point: 157-159°C
(Example 31) 2-(4-biphenylyl)-5-n-butyl-1,2,4-triazolo[1,5-c]quinazoline
   Melting point: 159 - 161°C
(Example 32) 5-n-butyl-2-(4-hydroxyphenyl)-1,2,4-triazolo[1,5-c]quinazoline
   Melting point: 234 - 235.5°C
(Example 33) 5-n-butyl-2-(2-methoxyphenyl)-1,2,4-triazolo[1,5-c]quinazoline
   Melting point: 1065 - 107.5°C
(Example 34) 5-n-butyl-2-(4-methoxyphenyl)-1,2,4-triazolo[1,5-c]quinazoline
   Melting point: 126 - 128°C
(Example 35) 5-n-butyl-2-(4-ethoxyphenyl)-1,2,4-triazolo[1,5-c]quinazoline
   Melting point: 149 - 150°C
(Example 36) 5-n-butyl-2-(4-propoxyphenyl)-1,2,4-triazolo[1,5-c]quinazoline
   Melting point: 128 - 129°C
(Example 37) 5-n-butyl-2-(3,4,5-trimethoxyphenyl)-1,2,4-triazolo[1,5-c]quinazoline
   Melting point: 130 - 133°C
(Example 38) 5-n-butyl-2-[4-(N,N-dimethylamino)phenyl)-1,2,4-triazolo[1,5-c]quinazoline
   Melting point: 1485 - 150°C
(Example 39) 5-n-butyl-2-(4-nitrophenyl)-1,2,4-triazolo[1,5-c]quinazoline
   Melting point: 194 - 195°C
(Example 40) 5-n-butyl-2-(3-pyridyl)-1,2,4-triazolo[1,5-c]quinazoline
   Melting point: 140 - 141.5°C
(Example 41) 5-n-butyl-2-(2-furyl)-1,2,4-triazolo[1,5-c]quinazoline
   Melting point: 110- 111.5°C
(Example 42) 5-n-butyl-2-(2-thienyl)-1,2,4-triazolo[1,5-c]quinazoline
   Melting point: 147 - 149.5°C
(Example 43) 5-n-butyl-10-fluoro-2-phenyl-1,2,4-triazolo[1,5-c]quinazoline
   Melting point: 123 - 124°C
(Example 44) 5-n-butyl-10-chloro-2-phenyl-1,2,4-triazolo[1,5-c]quinazoline
   Melting point: 122 - 123°C
(Example 45) 5-n-butyl-9-chloro-2-phenyl-1,2,4-triazolo[1,5-c]quinazoline
   Melting point: 110 - 113°C
(Example 46) 5-n-butyl-8-chloro-2-phenyl-1,2,4-triazolo[1,5-c]quinazoline
   Melting point: 143 - 144°C
(Example 47) 9-bromo-5-n-butyl-2-phenyl-1,2,4-triazolo[1,5-c]quinazoline
   Melting point: 1295 - 132.5°C
(Example 48) 5-n-butyl-8,9-dimethoxy-2-phenyl-1,2,4-triazolo[1,5-c]quinazoline
   Melting point: 150 - 152°C
(Example 49) 5-n-butyl-10-methyl-2-phenyl-1,2,4-triazolo[1,5-c]quinazoline
   Melting point: 123 - 124°C
(Example 50) 5-n-butyl-8-methyl-2-phenyl-1,2,4-triazolo[1,5-c]quinazoline
   Melting point: 1365 - 138°C
(Example 51) 5-n-butyl-2-(2-chlorophenyl)pyrazolo[4,3-e]-1,2,4-triazolo[1,5-c]pyrimidine
   Melting point: 206 - 207°C
(Example 52) 5-n-butyl-2-(3-chlorophenyl)pyrazolo[4,3-e]-1,2,4-triazolo[1,5-c]pyrimidine
   Melting point: 240 - 242.5°C
(Example 53) 5-n-butyl-2-(2-methoxyphenyl)pyrazolo[4,3-e]-1,2,4-triazolo[1,5-c]pyrimidine
   Melting point: 157 - 160°C
(Example 54) 5-n-butyl-2-(3-methoxyphenyl)pyrazolo[4,3-e]-1,2,4-triazolo[1,5-c]pyrimidine
   Melting point: 206 - 208°C
(Example 55) 5-n-butyl-2-(4-methoxyphenyl)pyrazolo[4,3-e]-1,2,4-triazolo[1,5-c]pyrimidine
   Melting point: 206 - 208°C
(Example 56) 5-n-butyl-2-(4-methylthiophenyl)pyrazolo[4,3-e]-1,2,4-triazolo[1,5-c]pyrimidine
   Melting point: 204 - 205°C
(Example 57) 5-n-butyl-2-(3-methoxyphenyl)-1,2,4-triazolo[1,5-c]quinazoline
   Melting point: 118 - 119°C
(Example 58) 5-n-butyl-2-(4-methylthiophenyl)-1,2,4-triazolo[1,5-c]quinazoline
   Melting point: 1405 - 141.5°C
(Example 59) 2,5-diphenylpyrazolo[4,3-e]-1,2,4-triazolo[1,5-c]pyrimidine
   Melting point: 2725 - 275°C
(Example 60) 2-(4-methylphenyl)-5-n-propylpyrazolo[4,3-e]-1,2,4-triazolo[1,5-c]pyrimidine
   Melting point: 274 - 275°C
(Example 61) 5-n-butyl-2-(4-ethylphenyl)pyrazolo[4,3-e]-1,2,4-triazolo[1,5-c]pyrimidine
   Melting point: 208.5 - 210°C
(Example 62) 5-n-butyl-2-(4-n-propylphenyl)pyrazolo[4,3-e]-1,2,4-triazolo[1,5-c]pyrimidine
   Melting point: 2075 - 209°C
(Example 63) 5-ethyl-2-(4-methoxyphenyl)pyrazolo[4,3-e]-1,2,4-triazolo[1,5-c]pyrimidine
   Melting point: 274 - 277°C
(Example 64) 2-(4-chlorophenyl)-5-ethylpyrazolo[4,3-e]-1,2,4-triazolo[1,5-c]pyrimidine
   Melting point: > 280°C
(Example 65) 5-ethyl-2-(4-methylphenyl)pyrazolo[4,3-e]-1,2,4-triazolo[1,5-c]pyrimidine
   Melting point: > 280°C
(Example 66) 2-(4-bromophenyl)-5-ethyl-1,2,4-triazolo[1,5-c]quinazoline
   Melting point: 197 - 198°C
(Example 67) 5-ethyl-2-(4-fluorophenyl)-1,2,4-triazolo[1,5-c]quinazoline
   Melting point: 195 - 196°C
(Example 68) 2-(4-chlorophenyl)-5-ethyl-1,2,4-triazolo[1,5-c]quinazoline
   Melting point: 197 - 198°C
(Example 69) 5-ethyl-2-(4-methylphenyl)-1,2,4-triazolo[1,5-c]quinazoline
   Melting point: 147 - 148°C
(Example 70) 5-ethyl-2-(4-hydroxyphenyl)-1,2,4-triazolo[1,5-c]quinazoline
   Melting point: > 280°C
(Example 71) 5-ethyl-2-(4-methoxyphenyl)-1,2,4-triazolo[1,5-c]quinazoline
   Melting point: 1675 - 168°C:
(Example 72) 2-(4-ethoxyphenyl)-5-ethyl-1,2,4-triazolo[1,5-c]quinazoline
   Melting point: 153 - 154°C
(Example 73) 9-chloro-2-phenyl-5-n-propyl-1,2,4-triazolo[1,5-c]quinazoline
   Melting point: 1405 - 141.5°C
(Example 74) 5-n-butyl-9-chloro-2-(4-fluorophenyl)-1,2,4-triazolo[1,5-c]quinazoline
   Melting point: 1475 - 150°C
(Example 75) 5-n-butyl-9-chloro-2-(4-chlorophenyl)-1,2,4-triazolo[1,5-c]quinazoline
   Melting point: 1385 - 140°C
(Example 76) 2-(4-bromophenyl)-5-n-butyl-9-chloro-1,2,4-triazolo[1,5-c]quinazoline
   Melting point: 158 - 159.5°C
(Example 77) 5-n-butyl-9-chloro-2-(4-methylphenyl)-1,2,4-triazolo[1,5-c]quinazoline
   Melting point: 141 - 143°C
(Example 78) 5-n-butyl-9-chloro-2-(4-methoxyphenyl)-1,2,4-triazolo[1,5-c]quinazoline
   Melting point: 124 - 125°C
(Example 79) 8-chloro-2-phenyl-5-n-propyl-1,2,4-triazolo[1,5-c]quinazoline
   Melting point: 147 - 149°C
(Example 80) 5-n-butyl-8-chloro-2-(4-fluorophenyl)-1,2,4-triazolo[1,5-c]quinazoline
   Melting point: 185 5 - 186.5°C
(Example 81) 5-n-butyl-8-chloro-2-(4-chlorophenyl)-1,2,4-triazolo[1,5-c]quinazoline
   Melting point: 153.5 - 155°C
(Example 82) 2-(4-bromophenyl)-5-n-butyl-8-chloro-1,2,4-triazolo[1,5-c]quinazoline
   Melting point: 150 - 151°C
(Example 83) 5-n-butyl-8-chloro-2-(4-methylphenyl)-1,2,4-triazolo[1,5-c]quinazoline
   Melting point: 134 - 134.5°C
(Example 84) 5-n-butyl-8-chloro-2-(4-methoxyphenyl)-1,2,4-triazolo[1,5-c]quinazoline
   Melting point: 1385 - 139.5°C
(Example 85) 5-n-butyl-9-nitro-2-phenyl-1,2,4-triazolo[1,5-c]quinazoline
   Melting point: 2055 - 206°C
(Example 86) 9-chloro- 2-(4-chlorophenyl)-5-ethyl-1,2,4-triazolo[1,5-c]quinazoline
   Melting point: 213 - 214°C

### <Example 87>

### Preparation of 5-n-pentyl-2-phenylpyrazolo[4,3-e]-1,2,4-triazolo[1,5-c]pyrimidine

3 g of the compound (5-ethyl-2-phenylpyrazolo[4,3-e]-1,2,4-triazolo[1,5-c]pyrimidine) obtained in Example 2 was dissolved in a mixed solution of 6 ml of concentrated hydrochloric acid and 30 ml of ethanol, followed by heating at reflux for 20 minutes. To the reaction solution, 60 ml of water was added and, after cooling to room temperature, the pH of the solution was adjusted to 8 by adding 25% aqueous ammonia. The deposited crystal was collected by filtration to obtain 2.2 g of 3-(3-aminopyrazol-4-yl)-5-phenyl-1,2,4-triazole.

To a solution of 0.5 g of the crystal in 5 ml of pyridine, a solution of 0.89 g of hexanoyl chloride in 5 ml of dichloromethane was added dropwise at 0°C and, after stirring at 0°C for 10 minutes, then at room temperature for 30 minutes, the mixture was heated at reflux for 15 minutes. The reaction solution was cooled to room temperature and the deposited crystal was collected by filtration to obtain 0.45 g of 3-[3-(N-hexanoylamino)pyrazol-4-yl]-5-phenyl-1,2,4-triazole (250 - 251°C).

Subseqeuntly, 0.4 g of the crystal thus obtained was suspended in 3 ml of acetonitrile and 1.0 ml of diisopropylethylamine and 0.78 ml of chlorotrimethylsilane were added, followed by heating at reflux for 26 hours. The reaction solution was cooled to room temperature, diluted with chloroform and then washed in turn with water and saturated saline. After the solvent was distilled off, the deposited crystal was washed with heated 50% methanol to obtain 0.26 g of a desired compound as a crystal.
Melting point: 200 - 201°C

### <Examples 88 to 107>

In the same manner as in Example 87, the following compounds were prepared.
(Example 88) 5-n-hexyl-2-phenylpyrazolo[4,3-e]-1,2,4-triazolo[1,5-c]pyrimidine
   Melting point: 207 - 208°C
(Example 89) methyl 4-[2-phenylpyrazolo[4,3-e]-1,2,4-triazolo[1,5-c]pyrimidin-5-yl]butyrate
   Melting point: 186 - 187°C
(Example 90) methyl 4-[2-(4-methoxyphenyl)pyrazolo[4,3-e]-1,2,4-triazolo[1,5-c]pyrimidin-5-yl]butyrate
   Melting point: 1875 - 188.5°C
(Example 91) methyl 4-[2-(4-chlorophenyl)pyrazolo[4,3-e]-1,2,4-triazolo[1,5-c]pyrimidin-5-yl]butyrate
   Melting point: 1945 - 196.5°C
(Example 92) methyl 4-[2-(4-methylphenyl)pyrazolo[4,3-e]-1,2,4-triazolo[1,5-c]pyrimidin-5-yl]butyrate
   Melting point: 1965 - 197°C
(Example 93) 5-n-pentyl-2-phenyl-1,2,4-triazolo[1,5-c]quinazoline
   Melting point: 118 - 118.5°C
(Example 94) 5-n-hexyl-2-phenyl-1,2,4-triazolo[1,5-c]quinazoline
   Melting point: 92 - 93°C
(Example 95) 2-(4-fluorophenyl)-5-n-pentyl-1,2,4-triazolo[1,5-c]quinazoline
   Melting point: 1335 - 134°C
(Example 96) methyl 4-(2-phenyl-1,2,4-triazolo[1,5-c]quinazolin-5-yl)butyrate
   Melting point: 1185 - 119.5°C
(Example 97) methyl 4-[2-(4-bromophenyl)-1,2,4-triazolo[1,5-c]quinazolin-5-yl]butyrate
   Melting point: 125 - 126°C
(Example 98) methyl 4-[2-(4-fluorophenyl)-1,2,4-triazolo[1,5-c]quinazolin-5-yl]butyrate
   Melting point: 167 - 168°C
(Example 99) methyl 4-[2-(4-chlorophenyl)-1,2,4-triazolo[1,5-c]quinazolin-5-yl]butyrate
   Melting point: 128 - 129°C
(Example 100) methyl 4-[2-(4-methylphenyl)-1,2,4-triazolo[1,5-c]quinazolin-5-yl]butyrate
   Melting point: 1205 - 121.5°C
(Example 101) 2-(4-methylphenyl)-5-n-pentyl-1,2,4-triazolo[1,5-c]quinazoline
   Melting point: 1295 - 131°C
(Example 102) 2-(4-bromophenyl)-5-n-pentyl-1,2,4-triazolo[1,5-c]quinazoline
   Melting point: 133 - 134°C
(Example 103) 2-(4-chlorophenyl)-5-n-pentyl-1,2,4-triazolo[1,5-c]quinazoline
   Melting point: 118 - 119.5°C
(Example 104) methyl 4-[9-chloro-2-(4-chlorophenyl)-1,2,4-triazolo[1,5-c]quinazolin-5-yl]butyrate
   Melting point: 145 - 146.5°C
(Example 105) 2-(4-methoxyphenyl)-5-n-pentyl-1,2,4-triazolo[1,5-c]quinazoline
   Melting point: 123 - 125°C
(Example 106) 2-(4-ethoxyphenyl)-5-n-pentyl-1,2,4-triazolo[1,5-c]quinazoline
   Melting point: 116 - 117°C
(Example 107) 2-(4-hexanoyloxyphenyl)-5-n-pentyl-1,2,4-triazolo[1,5-c]quinazoline
   Melting point: 805 - 82°C

### <Examples 108 and 109>

### Preparation of 5-n-butyl-2-(4-fluorophenyl)-7-methylpyrazolo[4,3-e]-1,2,4-triazolo[1,5-c]pyrimidine (Example 108) and 5-n-butyl-2-(4-fluorophenyl)-8-methylpyrazolo[43-e]-1,2,4-triazolo[1,5-c]pyrimidine (Example 109)

0.60 g of the compound (5-n-butyl-2-(4-fluorophenyl)pyrazolo[4,3-e]-1,2,4-triazolo[1,5-c]pyrimidine) obtained in Example and 032 g of anhydrous potassium carbonate were suspended in 10 ml of DMF, followed by stirring at room temperature for one hours, then at 50°C for 15 minutes. The mixed solution was cooled to 0°C and 030 g of methyl iodide was added dropwise, followed by stirring at 0°C for one hours, then at room temperature for 16 hours. To the reaction solution, iced water was added and the deposited crystal was purified by silica gel column chromatography (as an eluent, chloroform was used and then a mixture of chloroform and methanol (50:1) was used) to obtain 036 g of a desired 7-methyl derivative (melting point: 150.5 - 151.5°C) from the first fraction and 0.19 g of a 8-methyl derivative (melting point: 2055 - 206.5°) from the second fraction.

### <Examples 110 to 146>

In the same manner as in Examples 108 and 109, the following compounds were prepared.
(Example 110) 5-n-butyl-7-methyl-2-phenylpyrazolo[4,3-e]-1,2,4-triazolo[1,5-c]pyrimidine
   Melting point: 172 - 173°C
(Example 111) 5-n-butyl-8-methyl-2-phenylpyrazolo[4,3-e]-1,2,4-triazolo[1,5-c]pyrimidine
   Melting point: 184 - 185°C
(Example 112) 5-n-butyl-2-phenyl-7-n-propylpyrazolo[4,3-e]-1,2,4-triazolo[1,5-c]pyrimidine
   Melting point: 100.5 - 101.5°C
(Example 113) 5-n-butyl-2-phenyl-8-n-propylpyrazolo[4,3-e]-1,2,4-triazolo[1,5-c]pyrimidine
   Melting point: 107 - 108°C
(Example 114) 5-n-butyl-7-ethyl-2-phenylpyrazolo[4,3-e]-1,2,4-triazolo[1,5-c]pyrimidine
   Melting point: 146 - 147°C
(Example 115) 5-n-butyl-8-ethyl-2-phenylpyrazolo[4,3-e]-1,2,4-triazolo[1,5-c]pyrimidine
   Melting point: 122.5 - 123°C
(Example 116) 7-benzyl-5-n-butyl-2-phenylpyrazolo[4,3-e]-1,2,4-triazolo[1,5-c]pyrimidine
   Melting point: 108 - 109°C
(Example 117) 8-benzyl-5-n-butyl-2-phenylpyrazolo[4,3-e]-1,2,4-triazolo[1,5-c]pyrimidine
   Melting point: 159 - 160°C
(Example 118) 2-(4-chlorophenyl)-5-n-butyl-7-methylpyrazolo[4,3-e]-1,2,4-triazolo[1,5-c]pyrimidine
   Melting point: 1475 - 148.5°C
(Example 119) 2-(4-chlorophenyl)-5-n-butyl-8-methylpyrazolo[4,3-e]-1,2,4-triazolo[1,5-c]pyrimidine
   Melting point: 2035 - 205°C
(Example 120) 5-n-butyl-7-methyl-2-(4-methylphenyl)pyrazolo[4,3-e]-1,2,4-triazolo[1,5-c]pyrimidine
   Melting point: 156 - 157°C
(Example 121) 5-n-butyl-8-methyl-2-(4-methylphenyl)pyrazolo[4,3-e]-1,2,4-triazolo[1,5-c]pyrimidine
   Melting point: 180 - 181°C
(Example 122) 5-n-butyl-2-(4-methylphenyl)-7-n-propylpyrazolo[4,3-e]-1,2,4-triazolo[1,5-c]pyrimidine
   Melting point: 113 - 113.5°C
(Example 123) 5-n-butyl-2-(4-methylphenyl)-8-n-propylpyrazolo[4,3-e]-1,2,4-triazolo[1,5-c]pyrimidine
   Melting point: 101.5 - 103.5°C
(Example 124) 5-n-butyl-2-(4-fluorophenyl)-7-n-propylpyrazolo[4,3-e]-1,2,4-triazolo[1,5-c]pyrimidine
   Melting point: 1055 - 106°C
(Example 125) 5-n-butyl-2-(4-fluorophenyl)-8-n-propylpyrazolo[4,3-e]-1,2,4-triazolo[1,5-c]pyrimidine
   Melting point: 1065 - 107.5°C
(Example 126) 5-n-butyl-2-(4-chlorophenyl)-7-n-propylpyrazolo[4,3-e]-1,2,4-triazolo[1,5-c]pyrimidine
   Melting point: 1315 - 132°C
(Example 127) 5-n-butyl-2-(4-chlorophenyl)-8-n-propylpyrazolo[4,3-e]-1,2,4-triazolo[1,5-c]pyrimidine
   Melting point: 137 - 138°C
(Example 128) ethyl 2-(5-n-butyl-2-phenylpyrazolo[4,3-e]-1,2,4-triazolo[1,5-c]pyrimidin-7-yl)acetate
   Melting point: 1315 - 132.5°C
(Example 129) ethyl 2-(5-n-butyl-2-phenylpyrazolo[4,3-e]-1,2,4-triazolo[1,5-c]pyrimidin-8-yl)acetate
   Melting point: 136 - 137°C
(Example 130) ethyl 2-[5-n-butyl-2-(4-fluorophenyl)pyrazolo[4,3-e]-1,2,4-triazolo[1,5-c]pyrimidin-7-yl]acetate
   Melting point: 1395 - 140.5°C
(Example 131) ethyl 2-[5-n-butyl-2-(4-fluorophenyl)pyrazolo[4,3-e]-1,2,4-triazolo[1,5-c]pyrimidin-8-yl]acetate
   Melting point: 164 - 165°C
(Example 132) 5-n-butyl-7-(2-hydroxyethyl)-2-phenylpyrazolo[4,3-e]-1,2,4-triazolo[1,5-c]pyrimidine
   Melting point: 132 - 133°C
(Example 133)5-n-butyl-8-(2-hydroxyethyl)-2-phenylpyrazolo[4,3-e]-1,2,4-triazolo[1,5-c]pyrimidine
   Melting point: 1605 - 162°C
(Example 134) 5-n-butyl-2-(4-fluorophenyl)-7-(2-hydroxyethyl)pyrazolo[4,3-e]-1,2,4-triazolo[1,5-c]pyrimidine
   Melting point: 1505 - 151.5°C
(Example 135) 5-n-butyl-2-(4-fluorophenyl)-8-(2-hydroxyethyl)pyrazolo[4,3-e]-1,2,4-triazolo[1,5-c]pyrimidine
   Melting point: 202 - 203°C
(Example 136) 5-n-butyl-7-(2-hydroxyethyl)-2-(4-methylphenyl)pyrazolo[4,3-e]-1,2,4-triazolo[1,5-c]pyrimidine
   Melting point: 134 - 136°C
(Example 137) 5-n-butyl-8-(2-hydroxyethyl)-2-(4-methylphenyl)pyrazolo[4,3-e]-1,2,4-triazolo[1,5-c]pyrimidine
   Melting point: 171 - 173°C
(Example 138) 5-n-butyl-2-(4-chlorophenyl)-7-(2-hydroxyethyl)pyrazolo[4,3-a]-1,2,4-triazolo[1,5-c]pyrimidine
   Melting point: 164 - 164.5°C
(Example 139) 5-n-butyl-2-(4-chlorophenyl)-8-(2-hydroxyethyl)pyrazolo[4,3-a]-1,2,4-triazolo[1,5-c]pyrimidine
   Melting point: 189 - 190.5°C
(Example 140) 5-n-butyl-7-(2-hydroxyethyl)-2-(4-methoxyphenyl)pyrazolo[4,3-e]-1,2,4-triazolo[1,5-c]pyrimidine
   Melting point: 1475 - 148.5°C
(Example 141) 5-n-butyl-8-(2-hydroxyethyl)-2-(4-methoxyphenyl)pyrazolo[4,3-a]-1,2,4-triazolo[1,5-c]pyrimidine
   Melting point: 178 - 179°C
(Example 142) 5-n-butyl-8-ethyl-2-(4-methoxyphenyl)pyrazolo[4,3-e]-1,2,4-triazolo[1,5-c]pyrimidine
   Melting point: 127 - 128°C
(Example 143) 5-n-butyl-8-ethyl-2-(4-methylphenyl)pyrazolo[4,3-e]-1,2,4-triazolo[1,5-c]pyrimidine
   Melting point: 128 - 129°C
(Example 144) 5-n-butyl-8-ethyl-2-(4-fluorophenyl)pyrazolo[4,3-e]-1,2,4-triazolo[1,5-c]pyrimidine
   Melting point: 146 - 147°C
(Example 145) 5-n-butyl-2-(4-chlorophenyl)-8-ethylpyrazolo[4,3-e]-1,2,4-triazolo[1,5-c]pyrimidine
   Melting point: 141 - 142°C
(Example 146) 5-n-butyl-2-(4-methoxyphenyl)-8-n-propylpyrazolo[4,3-e]-1,2,4-triazolo[1,5-c]pyrimidine
   Melting point: 112 - -112.5°C

### <Example 147>

### Preparation of 4-(2-phenylpyrazolo[4,3-e]-1,2,4-triazolo[1,5-c]pyrimidin-5-yl)butyric acid

0.15 g of the compound (methyl 4-[2-phenylpyrazolo[4,3-e]-1,2,4-triazolo[1,5-c]pyrimidin-5-yl]butyrate) obtained in Example 89 was suspended in 3 ml of ethanol and 0.89 ml of a 5% aqueous sodium hydroxide solution was added, followed by stirring at room temperature for 4 hours. The reaction solution was diluted with 10 ml of water and then acidified by adding hydrochloric acid. The deposited crystal was collected by filtration and then recrystallized from hydrous ethanol to obtain 0.13 g of a desired compound as a crystal (melting point: 2615 - 262.5°C).

### <Examples 148 to 157>

In the same manner as in Example 147, the following compounds were prepared.
(Example 148) 4-[2-(4-methoxyphenyl)pyrazolo[4,3-e]-1,2,4-triazolo[1,5-c]pyrimidin-5-yl]butyric acid
   Melting point: 2475 - 249°C
(Example 149) 4-[2-(4-chlorophenyl)pyrazolo[4,3-e]-1,2,4-triazolo[1,5-c]pyrimidin-5-yl]butyric acid
   Melting point: 2535 - 255.5°C
(Example 150) 4-[2-(4-methylphenyl)pyrazolo[43-e]-1,2,4-triazolo[1,5-c]pyrimidin-5-yl]butyric acid
   Melting point: 249 - 251°C
(Example 151) 2-(5-n-butyl-2-phenylpyrazolo[4,3-e]-1,2,4-triazolo[1,5-c]pyrimidin-7-yl)acetic acid
   Melting point: 271 - 272.5°C
(Example 152) 2-(5-n-butyl-2-phenylpyrazolo[4,3-e]-1,2,4-triazolo[1,5-c]pyrimidin-8-yl)acetic acid
   Melting point: > 281°C (with decomposition)
(Example 153) 4-(2-phenyl-1,2,4-triazolo[1,5-c]quinazolin-5-yl)butyric acid
   Melting point: 211 - 211.5°C
(Example 154) 4-[2-(4-bromophenyl)-1,2,4-triazolo[1,5-c]quinazolin-5-yl]butyric acid
   Melting point: 219 - 220°C
(Example 155) 4-[2-(4-fluorophenyl)-1,2,4-triazolo[1,5-c]quinazolin-5-yl]butyric acid
   Melting point: 225 - 226°C
(Example 156) 4-[2-(4-chlorophenyl)-1,2,4-triazolo[1,5-c]quinazolin-5-yl]butyric acid
   Melting point: 227 - 228°C
(Example 157) 2-(4-hydroxyphenyl)-5-n-pentyl-1,2,4-triazolo[1,5-c]quinazoline
   Melting point: 214 - 215°C

Data of ¹H-NMR spectrum (δ:ppm) of the compounds of the above respective Examples are shown below. Dimethyl sulfoxide-d6 (DMSO-d6) or chloroform-d (CDCl3) was used as a solvent in the measurement and tetramethylsilane was used as an internal reference.

### Example 1 DMSO-d6

2.97 (3H, s), 75-7.6 (3H, m), 8.2-83 (2H, m), 857 (1H, bs).

### Example 2 DMSO-d6

1.45 (3H, t, J=7.4), 337 (2H, q, J=7.4), 75-7.6 (3H, m), 8.2-8.3 (2H, m), 856 (1H, bs).

### Example 3 DMSO-d6

1.07 (3H, t, J=7.4), 1.9-2.1 (2H, m), 3.3-3.4 (2H, m), 75-7.7 (3H, m), 8.2-8.4 (2H, m), 856 (1H, bs).

### Example 4 DMSO-d6

0.99 (3H, t, J=7.2), 1.4-1.6 (2H, m), 1.9-2.0 (2H, m), 33-3.4 (2H, m), 75-7.6 (3H, m), 8.2-83 (2H, m), 856 (1H, bs).

### Example 5 DMSO-d6

0.98 (3H, t, J=7.4), 1.4-1.5 (2H, m), 1.9-2.0 (2H, m), 33-3.4 (2H, m), 7.40 (2H, t, J=8.8), 8.29 (2H, dd, J=5.5, 8.8), 8.55 (1H, bs).

### Example 6 DMSO-d6

0.98 (3H, t, J=7.4), 1.4-1.5 (2H, m), 1.8-2.0 (2H, m), 3.3-3.4 (2H, m), 7.75 (2H, d, J=8.5), 8.16 (2H, d, J=85), 854 (1H, bs).

### Example 7 DMSO-d6

0.98 (3H, t, J=73), 1.4-1.5 (2H, m), 1.9-2.0 (2H, m), 3.3-3.4 (2H, m), 7.63 (2H, d, J=8.6), 8.24 (2H, d, J=8.6), 8.55 (1H, bs).

### Example 8 DMSO-d6

0.98 (3H, t, J=73), 1.4-1.5 (2H, m), 1.9-2.0 (2H, m), 2.40 (3H, s), 33-3.4 (2H, m), 737 (2H, d, J=8.1), 8.14 (2H, d, J=8.1), 854 (1H, bs).

### Example 9 DMSO-d6

0.98 (3H, t, J=7.4), 134 (9H, s), 1.4-1.5 (2H, m), 1.9-2.0 (2H, m), 33-3.4 (2H, m), 758 (2H, d, J=8.5), 8.17 (2H, d, J=8.5), 8.55 (1H, bs).

### Example 10 DMSO-d6

0.99 (3H, t, J=73), 1.4-1.6 (2H, m), 1.9-2.0 (2H, m), 3.3-3.4 (2H, m), 7.91 (2H, d, J=83), 8.42 (2H, d, J=83), 8.56 (1H, bs).

### Example 11 DMSO-d6

0.99 (3H, t, J=7.3), 1.4-1.6 (2H, m), 1.9-2.0 (2H, m), 33-3.4 (2H, m), 7.4-7.6 (3H, m), 7.77 (2H, d, J=73), 7.87 (2H, d, J=8.4), 8.33 (2H, d, J=8.4), 857 (1H, bs).

### Example 12 DMSO-d6

0.98 (3H, t, J=73), 1.4-1.5 (2H, m), 1.9-2.0 (2H, m), 3.3-3.4 (2H, m), 6.94 (2H, d, J=8.7), 8.09 (2H, d, J=8.7), 852 (1H, bs).

### Example 13 DMSO-d6

0.98 (3H, t, J=7.4), 137 (3H, t, J=6.9), 1.4-1.5 (2H, m), 1.9-2.0 (2H, m), 3.3-3.4 (2H, m), 4.12 (2H, q, J=7.0), 7.08 (2H, d, J=8.9), 8.16 (2H, d, J=8.9), 853 (1H, bs).

### Example 14 DMSO-d6

0.98 (3H, t, J=75), 1.01 (3H, t, J=7.5), 1.4-1.6 (2H, m), 1.7-1.8 (2H, m), 1.9-2.0 (2H, m), 33-3.4 (2H, m), 4.01 (2H, t, J=6.6), 7.09 (2H, d, J=8.7), 8.16 (2H, d, J=8.7), 852 (1H, bs).

### Example 15 DMSO-d6

0.99 (3H, t, J=7.4), 1.4-1.5 (2H, m), 1.9-2.0 (2H, m), 33-3.4 (2H, m), 3.76 (3H, s), 3.91 (6H, s), 752 (2H, s), 856 (1H, bs).

### Example 16 DMSO-d6

0.98 (3H, t, J=73), 1.4-1.5 (2H, m), 1.9-2.0 (2H, m), 3.01 (6H, s), 3.3-3.4 (2H, m), 6.83 (2H, d, J=8.9), 8.06 (2H, d, J=8.9), 850 (1H, bs).

### Example 17 DMSO-d6

0.99 (3H, t, J=73), 1.4-1.5 (2H, m), 1.9-2.0 (2H, m), 33-3.4 (2H, m), 839 (2H, d, J=9.0), 8.46 (2H, d, J=9.0), 858 (1H, bs).

### Example 18 DMSO-d6

0.99 (3H, t, J=7.5), 1.4-1.5 (2H, m), 1.9-2.0 (2H, m), 3.3-3.4 (2H, m), 7.61 (1H, dd, J=5.0, 7.9), 85-8.6 (2H, m), 8.75 (1H, dd, J=1.7, 5.0), 9.40 (1H, d, J=2.1).

### Example 19 DMSO-d6

0.98 (3H, t, J=7.4), 1.4-1.5 (2H, m), 1.9-2.0 (2H, m), 3.3-3.4 (2H, m), 7.47 (1H, dd, J=1.8, 3.4), 7.28 (1H, dd, J=0.6, 3.4), 7.96 (1H, dd, J=0.6, 1.8), 853 (1H, bs).

### Example 20 DMSO-d6

0.98 (3H, t, J=75), 1.4-1.5 (2H, m), 1.9-2.0 (2H, m), 3.3-3.4 (2H, m), 7.25 (1H, dd, J=33, 5.0), 7.79 (1H, dd, J=1.2, 5.0), 7.89 (1H, dd, J=1.2, 33), 855 (1H, bs).

### Example 21 CDCl3

1.16 (3H, t, J=7.4), 2.0-2.1 (2H, m), 3.4-3.5 (2H, m), 75-7.6 (3H, m), 7.6-7.7 (1H, m), 7.8-7.9 (1H, m), 8.01 (1H, bd, J=8.2), 83-8.4 (2H, m), 858 (1H, dd, J=1.1, 8.0).

### Example 22 DMSO-d6

0.99 (3H, t, J=73), 1.4-1.6 (2H, m), 1.9-2.0 (2H, m), 3.3-3.4 (2H, m), 75-7.6 (3H, m), 7.7-7.8 (1H, m), 7.9-8.0 (1H, m), 8.02 (1H, bd, J=8.0), 8.2-83 (2H, m), 8.47 (1H, dd, J=0.9, 7.9).

### Example 23 CDCl3

1.04 (3H, t, J=73), 15-1.6 (2H, m), 2.0-2.1 (2H, m), 3.4-3.5 (2H, m), 7.21 (2H, t, J=8.7), 7.6-7.7 (1H, m), 7.8-7.9 (1H, m), 8.01 (1H, bd, J=8.2), 838 (2H, dd, J=5.4, 8.7), 8.55 (1H, dd, J=1.1, 8.0).

### Example 24 CDCl3

1.03 (3H, t, J=73), 15-1.6 (2H, m), 2.0-2.1 (2H, m), 3.4-35 (2H, m), 7.4-7.5 (2H, m), 75-7.6 (1H, m), 7.6-7.7 (1H, m), 7.8-7.9 (1H, m), 8.03 (1H, bd, J=8.2), 8.0-8.1 (1H, m), 857 (1H, dd, J=1.5, 7.9).

### Example 25 CDCl3

1.05 (3H, t, J=7.6), 15-1.6 (2H, m), 2.0-2.1 (2H, m), 3.4-3.5 (2H, m), 7.4-7.5 (2H, m), 7.7-7.8 (1H, m), 7.8-7.9 (1H, m), 8.01 (1H, bd, J=8.5), 8.2-83 (1H, m), 839 (1H, brs), 855 (1H, dd, J=1.5, 7.9).

### Example 26 CDCl3

1.04 (3H, t, J=7.4), 15-1.6 (2H, m), 2.0-2.1 (2H, m), 3.4-35 (2H, m), 750 (2H, d, J=85), 7.6-7.7 (1H, m), 7.7-7.8 (1H, m), 8.01 (1H, bd, J=8.2), 833 (2H, d, J=85), 855 (1H, dd, J=0.9, 7.8).

### Example 27 CDCl3

1.04 (3H, t, J=7.4), 1.5-1.6 (2H, m), 1.9-2.0 (2H, m), 3.4-3.5 (2H, m), 7.66 (2H, d, J=8.6), 7.6-7.7 (1H, m), 7.7-7.8 (1H, m), 8.01 (1H, bd, J=8.1), 8.26 (2H, d, J=8.6), 855 (1H, dd, J=1.1, 8.1).

### Example 28 CDCl3

1.04 (3H, t, J=73), 1.5-1.6 (2H, m), 2.0-2.1 (2H, m), 3.4-3.5 (2H, m), 733 (2H, d, J=8.0), 7.6-7.7 (1H, m), 7.7-7.8 (1H, m), 8.00 (1H, bd, J=8.2), 8.27 (2H, d, J=8.0), 856 (1H, dd, J=1.0, 7.9).

### Example 29 CDCl3

1.04 (3H, t, J=73), 139 (9H, s), 15-1.6 (2H, m), 2.0-2.1 (2H, m), 3.4-3.5 (2H, m), 7.55 (2H, d, J=85), 7.6-7.7 (1H, m), 7.7-7.8 (1H, m), 8.00 (1H, bd, J=8.2), 830 (2H, d, J=85), 8.57 (1H, dd, J=13, 7.9).

### Example 30 CDCl3

1.05 (3H, t, J=7.5), 1.5-1.6 (2H, m), 2.0-2.1 (2H, m), 3.4-3.5 (2H, m), 7.6-7.7 (1H, m), 7.77 (2H, d, J=7.9), 7.8-7.9 (1H, m), 8.01 (1H, bd, J=83), 8.49 (2H, d, J=7.9), 855 (1H, dd, J=1.2, 7.9).

### Example 31 CDCl3

1.05 (3H, t, J=75), 1.5-1.6 (2H, m), 2.0-2.1 (2H, m), 3.4-3.5 (2H, m), 7.3-7.4 (1H, m), 7.4-75 (2H, m), 7.6-7.7 (3H, m),7.76 (2H, d, J=7.9), 7.7-7.8 (1H, m), 8.01 (1H, bd, J=83), 8.45 (2H, d, J=7.9), 858 (1H, dd, J=1.2, 7.9).

### Example 32 DMSO-d6

0.99 (3H, t, J=7.4), 1.4-1.5 (2H, m), 1.9-2.0 (2H, m), 3.3-3.4 (2H, m), 6.96 (2H, d, J=8.6), 7.7-7.8 (1H, m), 7.8-7.9 (1H, m), 7.99 (1H, bd, J=8.2), 8.12 (2H, d, J=8.6), 8.43 (1H, bd, J=7.9).

### Example 33 CDCl3

1.03 (3H, t, J=73), 1.5-1.6 (2H, m), 2.0-2.1 (2H, m), 3.4-35 (2H, m), 3.98 (3H, s), 7.1-7.2 (2H, m), 7.4-7.5 (1H, m), 7.67 (1H, bt, J=7.9), 7.7-7.8 (1H, m), 8.01 (1H, bd, J=8.2), 8.06 (1H, dd, J=1.8, 7.6), 859 (1H, bd, J=7.9).

### Example 34 CDCl3

1.04 (3H, t, J=7.4), 15-1.6 (2H, m), 2.0-2.1 (2H, m), 3.4-3.5 (2H, m), 3.90 (3H, s), 7.04 (2H, d, J=8.9), 7.6-7.7 (1H, m), 7.7-7.8 (1H, m), 7.99 (1H, d, J=8.1), 832 (2H, bd, J=8.9), 856 (1H, dd, J=1.1, 7.9).

### Example 35 CDCl3

1.04 (3H, t, J=73), 1.47 (3H, t, J=7.0), 15-1.6 (2H, m), 2.0-2.1 (2H, m), 3.4-3.5 (2H, m), 4.13 (2H, q, J=7.0), 7.03 (2H, d, J=8.9), 7.6-7.7 (1H, m), 7.7-7.8 (1H, m), 7.99 (1H, bd, J=8.2), 8.31 (2H, d, J=8.9), 855 (1H, dd, J=1.0, 8.0).

### Example 36 CDCl3

1.04 (3H, t, J=73), 1.08 (3H, t, J=7.6), 15-1.6 (2H, m), 1.8-1.9 (2H, m), 2.0-2.1 (2H, m), 3.4-35 (2H, m), 4.01 (2H, t, J=6.4), 7.03 (2H, d, J=8.8), 7.6-7.7 (1H, m), 7.7-7.8 (1H, m), 7.99 (1H, bd, J=8.2), 830 (2H, d, J=8.8), 855 (1H, dd, J=1.5, 7.9).

### Example 37 CDCl3

1.05 (3H, t, J=73), 15-1.6 (2H, m), 2.0-2.1 (2H, m), 3.4-3.5 (2H, m), 3.94 (3H, s), 4.04 (6H, s), 7.64 (2H, s), 7.6-7.7 (1H, m), 7.8-7.9 (1H, m), 8.01 (1H, bd, J=8.2), 857 (1H, dd, J=1.5, 7.9).

### Example 38 CDCl3

1.04 (3H, t, J=73), 15-1.6 (2H, m), 2.0-2.1 (2H, m), 3.06 (6H, s), 3.4-35 (2H, m), 6.81 (2H, d, J=9.1), 7.6-7.7 (1H, m), 7.7-7.8 (1H, m), 7.97 (1H, bd, J=8.2), 8.24 (2H, d, J=9.1), 855 (1H, dd, J=1.5, 7.9).

### Example 39 CDCl3

1.05 (3H, t, J=7.4), 15-1.6 (2H, m), 2.0-2.1 (2H, m), 3.4-3.5 (2H, m), 7.7-7.8 (1H, m), 7.8-7.9 (1H, m), 8.04 (1H, bd, J=8.1), 838 (2H, d, J=9.0), 85-8.6 (1H, m), 858 (2H, d, J=9.0).

### Example 40 CDCl3

1.05 (3H, t, J=73), 1.5-1.6 (2H, m), 2.0-2.1 (2H, m), 3.4-3.5 (2H, m), 7.46 (1H, dd, J=5.0, 7.9), 7.6-7.7 (1H, m), 7.8-7.9 (1H, m), 8.02 (1H, bd, J=8.2), 856 (1H, dd, J=15, 7.9), 8.64 (1H, dt, J=7.9, 2.1), 8.74 (1H, dd, J=2.1, 5.0), 959 (1H, bd, J=2.1).

### Example 41 CDCl3

1.03 (3H, t, J=73), 1.5-1.6 (2H, m), 2.0-2.1 (2H, m), 3.4-3.5 (2H, m), 6.61 (1H, dd, J=1.8, 3.2), 730 (1H, dd, J=0.6, 3.2), 7.66 (1H, dd, J=0.6, 1.8), 7.6-7.7 (1H, m), 7.8-7.9 (1H, m), 8.01 (1H, bd, J=85), 856 (1H, dd, J=1.5, 7.9).

### Example 42 CDCl3

1.04 (3H, t, J=7.6), 15-1.6 (2H, m), 2.0-2.1 (2H, m), 3.3-3.4 (2H, m), 7.19 (1H, dd, J=3.8, 53), 7.49 (1H, dd, J=1.2, 53), 7.6-7.7 (1H, m), 7.7-7.8 (1H, m), 7.9-8.0 (2H, m), 855 (1H, dd, J=1.5, 7.9).

### Example 43 CDCl3

1.05 (3H, t, J=73), 15-1.6 (2H, m), 2.0-2.1 (2H, m), 3.4-35 (2H, m), 7.40 (1H, bt, J=8.2), 75-7.6 (3H, m), 7.75 (1H, dt, J=5.6, 8.2), 7.82 (1H, bd, J=8.2), 8.4-85 (2H, m).

### Example 44 CDCl3

1.04 (3H, t, J=73), 15-1.6 (2H, m), 2.0-2.1 (2H, m), 3.4-3.5 (2H, m), 75-7.6 (3H, m), 7.68 (1H, bt, J=7.9), 7.72 (1H, dd, J=1.8, 7.9), 7.93 (1H, dd, J=1.8, 7.9),8.4-8.5 (2H, m).

### Example 45 CDCl3

1.04 (3H, t, J=7.3), 1.5-1.6 (2H, m), 2.0-2.1 (2H, m), 3.4-3.5 (2H, m), 75-7.6 (3H, m), 7.73 (1H, dd, J=2.6, 8.8), 7.93 (1H, d, J=8.8), 83-8.4 (2H, m), 855 (1H, d, J=2.6).

### Example 46 CDCl3

1.04 (3H, t, J=73), 15-1.6 (2H, m), 2.0-2.1 (2H, m), 3.4-35 (2H, m), 75-7.6 (3H, m), 7.64 (1H, dd, J=2.0, 8.8), 8.01 (1H, d, J=2.0), 8.3-8.4 (2H, m), 8.49 (1H, d, J=8.8).

### Example 47 CDCl3

1.04 (3H, t, J=73), 15-1.6 (2H, m), 2.0-2.1 (2H, m), 3.3-3.4 (2H, m), 75-7.6 (3H, m), 7.8-7.9 (2H, m), 83-8.4 (2H, m), 8.7-8.8 (1H, m).

### Example 48 CDCl3

1.04 (3H, t, J=73), 1.5-1.6 (2H, m), 2.0-2.1 (2H, m), 3.3-3.4 (2H, m), 4.06 (3H, s), 4.12 (3H, s), 7.41 (1H, s), 75-7.6 (3H, m), 7.86 (1H, s), 83-8.4 (2H, m).

### Example 49 CDCl3

1.04 (3H, t, J=73), 15-1.6 (2H, m), 2.0-2.1 (2H, m), 3.18 (3H, s), 3.4-35 (2H, m), 7.4-7.6 (4H, m), 7.66 (1H, bt, J=7.9), 7.84 (1H, bd, J=7.9), 8.4-8.5 (2H, m).

### Example 50 CDCl3

1.04 (3H, t, J=73), 15-1.6 (2H, m), 2.0-2.1 (2H, m), 258 (3H, s), 3.4-35 (2H, m), 75-7.6 (4H, m), 7.81 (1H, bs), 83-8.4 (2H, m), 8.44 (1H, d, J=8.2).

### Example 51 DMSO-d6

0.97 (3H, t, J=75), 1.4-1.5 (2H, m), 1.9-2.0 (2H, m), 3.3-3.4 (2H, m), 75-7.6 (2H, m), 7.6-7.7 (1H, m), 8.08 (1H, dd, J=25, 7.1), 857 (1H, bs).

### Example 52 DMSO-d6

0.99 (3H, t, J=7.5), 1.4-1.5 (2H, m), 1.9-2.0 (2H, m), 3.3-3.4 (2H, m), 75-7.6 (2H, m), 8.1-8.2 (2H, m), 852 (1H, bs).

### Example 53 DMSO-d6

0.98 (3H, t, J=75), 1.4-1.5 (2H, m), 1.9-2.0 (2H, m), 33-3.4 (2H, m), 3.88 (3H, s), 7.11 (1H, bt, J=75), 7.22 (1H, bd, J=8.7), 752 (1H, m), 7.93 (1H, dd, J=1.7, 75), 853 (1H, bs).

### Example 54 DMSO-d6

0.99 (3H, t, J=75), 1.4-1.5 (2H, m), 1.9-2.0 (2H, m), 3.3-3.4 (2H, m), 3.87 (3H, s), 7.11 (1H, dd, J=25, 7.9), 7.48 (1H, bt, J=7.9), 7.7-7.8 (1H, m), 7.84 (1H, bd, J=7.9), 856 (1H, bs).

### Example 55 DMSO-d6

0.98 (3H, t, J=75), 1.4-15 (2H, m), 1.9-2.0 (2H, m), 33-3.4 (2H, m), 3.85 (3H, s), 7.10 (2H, d, J=9.1), 8.17 (2H, d, J=9.1), 852 (1H, bs).

### Example 56 DMSO-d6

0.98 (3H, t, J=7.5), 1.4-1.5 (2H, m), 1.9-2.0 (2H, m), 255 (3H, s), 33-3.4 (2H, m), 7.41 (2H, d, J=8.7), 8.15 (2H, d, J=8.7), 853 (1H, bs).

### Example 57 CDCl3

1.04 (3H, t, J=73), 15-1.6 (2H, m), 2.0-2.1 (2H, m), 3.4-3.5 (2H, m), 3.95 (3H, s), 7.05 (1H, dd, J=2.6, 7.9), 7.44 (1H, t, J=7.9), 7.6-7.7 (1H, m), 7.8-7.9 (1H, m), 7.9-8.0 (1H, m), 8.0-8.1 (2H, m), 857 (1H, dd, J=1.5, 8.2).

### Example 58 CDCl3

1.04 (3H, t, J=73), 15-1.6 (2H, m), 2.0-2.1 (2H, m), 256 (3H, s), 3.4-3.5 (2H, m), 737 (2H, d, J=8.5), 7.6-7.7 (1H, m), 7.7-7.8 (1H, m), 8.00 (1H, bd, J=8.2), 8.29 (2H, d, J=85), 8.56 (1H, dd, J=1.5, 7.9).

### Example 59 DMSO-d6

75-7.6 (3H, m), 7.6-7.7 (3H, m), 8.2-83 (2H, m), 8.4-8.5 (2H, m), 8.65 (1H, bs).

### Example 60 DMSO-d6

1.07 (3H, t, J=7.5), 1.9-2.0 (2H, m), 2.40 (3H, s), 3.3-3.4 (2H, m), 737 (2H, d, J=83), 8.14 (2H, d, J=8.3), 8.54 (1H, bs).

### Example 61 CDCl3

1.05 (3H, t, J=7.5), 1.30 (3H, t, J=7.5), 1.5-1.6 (2H, m), 2.0-2.1 (2H, m), 2.74 (2H, q, J=75), 3.3-3.4 (2H, m), 735 (2H, d, J=83), 8.26 (2H, d, J=83), 8.47 (1H, s).

### Example 62 CDCl3

0.98 (3H, t, J=75), 1.05 (3H, t, J=75), 1.5-1.6 (2H, m), 1.6-1.7 (2H, m), 2.0-2.1 (2H, m), 2.6-2.7 (2H, m), 3.4-3.5 (2H, m), 733 (2H, d, J=83), 8.25 (2H, d, J=83), 8.47 (1H, s).

### Example 63 DMSO-d6

1.45 (3H, t, J=7.5), 336 (2H, q, J=75), 3.85 (3H, s), 7.10 (2H, d, J=8.7), 8.18 (2H, d, J=8.7), 8.53 (1H, bs).

### Example 64 DMSO-d6

1.44 (3H, t, J=75), 335 (2H, q, J=75), 7.61 (2H, d, J=83), 8.22 (2H, d, J=83), 854 (1H, bs).

### Example 65 DMSO-d6

1.44 (3H, t, J=7.5), 239 (3H, s), 336 (2H, q, J=75), 735 (2H, d, J=83), 8.12 (2H, d, J=83), 8.53 (1H, bs).

### Example 66 CDCl3

158 (3H, t, J=7.6), 3.45 (2H, q, J=7.6), 7.65 (2H, d, J=85), 7.6-7.7 (1H, m), 7.8-7.9 (1H, m), 8.02 (1H, bd, J=8.2), 8.25 (2H, d, J=85), 8.5-8.6 (1H, m).

### Example 67 CDCl3

159 (3H, t, J=73), 3.45 (2H, q, J=73), 7.21 (2H, t, J=8.8), 7.6-7.7 (1H, m), 7.8-7.9 (1H, m), 8.02 (1H, bd, J=8.2), 8.38 (2H, dd, J=5.6, 8.8), 856 (1H, dd, J=1.5, 7.9).

### Example 68 CDCl3

158 (3H, t, J=73), 3.44 (2H, q, J=73), 7.49 (2H, d, J=8.5), 7.68 (1H, bt, J=73), 7.8-7.9 (1H, m), 8.02 (1H, bd, J=8.2), 832 (2H, d, J=8.5), 85-8.6 (1H, m).

### Example 69 CDCl3

159 (3H, t, J=7.6), 2.44 (3H, s), 3.46 (2H, q, J=7.6), 733 (2H, d, J=8.5), 7.6-7.7 (1H, m), 7.8-7.9 (1H, m), 8.01 (1H, bd, J=8.2), 8.27 (2H, d, J=85), 857 (1H, dd, J=1.5, 7.9).

### Example 70 DMSO-d6

1.48 (3H, t, J=75), 336 (2H, q, J=75), 6.95 (2H, d, J=8.7), 7.7-7.8 (1H, m), 7.8-7.9 (1H, m), 8.00 (1H, bd, J=83), 8.12 (2H, d, J=8.7), 8.44 (1H, dd, J=1.2, 7.9).

### Example 71 CDCl3

158 (3H, t, J=75), 3.44 (2H, q, J=75), 3.89 (3H, s), 7.03 (2H, d, J=8.7), 7.6-7.7 (1H, m), 7.7-7.8 (1H, m), 8.00 (1H, bd, J=83), 831 (2H, d, J=8.7), 8.55 (1H, dd, J=1.3, 7.5).

### Example 72 CDCl3

1.47 (3H, t, J=7.0), 158 (3H, t, J=7.6), 3.45 (2H, q, J=7.6), 4.13 (2H, q, J=7.0), 7.03 (2H, d, J=8.8), 7.6-7.7 (1H, m), 7.7-7.8 (1H, m), 8.01 (1H, bd, J=7.9), 830 (2H, d, J=8.8), 856 (1H, dd, J=1.2, 8.2).

### Example 73 CDCl3

1.15 (3H, t, J=73), 2.0-2.1 (2H, m), 33-3.4 (2H, m), 75-7.6 (3H, m), 7.74 (1H, dd, J=23, 8.8), 7.94 (1H, d, J=8.8), 83-8.4 (2H, m), 855 (1H, d, J=23).

### Example 74 CDCl3

1.04 (3H, t, J=73), 15-1.6 (2H, m), 2.0-2.1 (2H, m), 33-3.4 (2H, m), 7.21 (2H, t, J=8.8), 7.73 (1H, dd, J=23, 8.8), 7.93 (1H, d, J=8.8), 836 (2H, dd, J=5.6, 8.8), 852 (1H, d, J=23).

### Example 75 CDCl3

1.04 (3H, t, J=73), 15-1.6 (2H, m), 2.0-2.1 (2H, m), 33-3.4 (2H, m), 7.49 (2H, d, J=8.8), 7.73 (1H, dd, J=23, 8.8), 7.93 (1H, d, J=8.8), 8.29 (2H, d, J=8.8), 8.51 (1H, d, J=2.3).

### Example 76 CDCl3

1.04 (3H, t, J=73), 15-1.6 (2H, m), 2.0-2.1 (2H, m), 33-3.4 (2H, m), 7.65 (2H, d, J=8.5), 7.73 (1H, dd, J=23, 8.8), 7.93 (1H, d, J=8.8), 8.23 (2H, d, J=8.5), 8.51 (1H, d, J=23).

### Example 77 CDCl3

1.04 (3H, t, J=73), 15-1.6 (2H, m), 2.0-2.1 (2H, m), 2.45 (3H, s), 3.4-3.5 (2H, m), 733 (2H, d, J=8.2), 7.72 (1H, dd, J=23, 8.8), 7.92 (1H, d, J=8.8), 8.25 (2H, d, J=8.2), 854 (1H, d, J=2.3).

### Example 78 CDCl3

1.04 (3H, t, J=73), 15-1.6 (2H, m), 2.0-2.1 (2H, m), 3.3-3.4 (2H, m), 3.90 (3H, s), 7.04 (2H, d, J=8.8), 7.72 (1H, dd, J=23, 8.8), 7.92 (1H, d, J=8.8), 830 (2H, d, J=8.8), 853 (1H, d, J=2.3).

### Example 79 CDCl3

1.15 (3H, t, J=73), 2.0-2.1 (2H, m), 33-3.4 (2H, m), 7.5-7.6 (3H, m), 7.64 (1H, dd, J=2.0, 8.5), 8.02 (1H, d, J=2.0), 83-8.4 (2H, m), 8.50 (1H, d, J=8.5).

### Example 80 CDCl3

1.04 (3H, t, J=7.6), 1.5-1.6 (2H, m), 2.0-2.1 (2H, m), 3.4-3.5 (2H, m), 7.21 (2H, t, J=8.8), 7.64 (1H, dd, J=2.1, 8.5), 8.02 (1H, d, J=2.1), 836 (2H, dd, J=5.3, 8.8), 8.48 (1H, d, J=8.5).

### Example 81 CDCl3

1.04 (3H, t, J=73), 1.5-1.6 (2H, m), 2.0-2.1 (2H, m), 33-3.4 (2H, m), 7.49 (2H, d, J=85), 7.63 (1H, dd, J=2.0, 8.5), 8.00 (1H, d, J=2.0), 8.29 (2H, d, J=85), 8.45 (1H, d, J=85).

### Example 82 CDCl3

1.04 (3H, t, J=73), 15-1.6 (2H, m), 2.0-2.1 (2H, m), 3.3-3.4 (2H, m), 7.6-7.7 (1H, m), 7.65 (2H, d, J=8.2), 8.02 (1H, d, J=1.8), 8.24 (2H, d, J=8.2), 8.47 (1H, d, J=8.8).

### Example 83 CDCl3

1.04 (3H, t, J=73), 1.5-1.6 (2H, m), 2.0-2.1 (2H, m), 2.45 (3H, s), 3.4-3.5 (2H, m), 733 (2H, d, J=8.2), 7.63 (1H, dd, J=1.8, 8.5), 8.00 (1H, d, J=1.8), 8.25 (2H, d, J=8.2), 8.49 (1H, d, J=8.5).

### Example 84 CDCl3

1.04 (3H, t, J=73), 15-1.6 (2H, m), 2.0-2.1 (2H, m), 3.3-3.4 (2H, m), 3.90 (3H, s), 7.04 (2H, d, J=8.8), 7.62 (1H, dd, J=2.0, 85), 8.00 (1H, d, J=2.0), 8.29 (2H, d, J=8.8), 8.47 (1H, d, J=8.5).

### Example 85 CDCl3

1.06 (3H, t, J=7.3), 15-1.6 (2H, m), 2.0-2.1 (2H, m), 3.4-3.5 (2H, m), 7.5-7.6 (3H, m), 8.14 (1H, d, J=9.1), 83-8.4 (2H, m), 859 (1H, dd, J=2.6, 9.1), 9.47 (1H, d, J=2.6).

### Example 86 CDCl3

1.57 (3H, t, J=7.6), 3.43 (2H, q, J=7.6), 7.49 (2H, d, J=8.2), 7.74 (1H, dd, J=23, 8.8), 7.95 (1H, d, J=8.8), 830 (2H, d, J=8.2), 852 (1H, d, J=23).

### Example 87 DMSO-d6

0.92 (3H, t, J=7.1), 1.4-1.5 (4H, m), 1.9-2.0 (2H, m), 3.3-3.4 (2H, m), 7.5-7.6 (3H, m), 8.2-83 (2H, m), 85.6 (1H, bs).

### Example 88 CDCl3

0.92 (3H, t, J=7.1), 13-15 (4H, m), 1.5-1.6 (2H, m), 2.0-2.1 (2H, m), 3.4-3.5 (2H, m), 75-7.6 (3H, m), 8.3-8.4 (2H, m), 8.46 (1H, s).

### Example 89 DMSO-d6

2.2-23 (2H, m), 258 (2H, t, J=75), 3.41 (2H, t, J=75), 358 (3H, s), 75-7.6 (3H, m), 8.2-83 (2H, m), 8.57 (1H, bs).

### Example 90 DMSO-d6

2.2-23 (2H, m), 257 (2H, t, J=7.5), 338 (2H, t, J=7.5), 357 (3H, s), 3.85 (3H, s), 7.11 (2H, d, J=9.1), 8.18 (2H, d, J=9.1), 853 (1H, bs).

### Example 91 DMSO-d6

2.2-23 (2H, m), 257 (2H, t, J=7.5), 339 (2H, t, J=7.5), 357 (3H, s), 7.63 (2H, d, J=83), 8.25 (2H, d, J=83), 855 (1H, bs).

### Example 92 DMSO-d6

2.2-23 (2H, m), 2.40 (3H, s), 257 (2H, t, J=75), 339 (2H, t, J=7.5), 357 (3H, s), 737 (2H, d, J=7.9), 8.14 (2H, d, J=7.9), 854 (1H, bs).

### Example 93 CDCl3

0.96 (3H, t, J=7.1), 1.4-1.6 (4H, m), 2.0-2.1 (2H, m), 3.4-3.5 (2H, m), 75-7.6 (3H, m), 7.6-7.7 (1H, m), 7.7-7.8 (1H, m), 8.00 (1H, bd, J=83), 8.3-8.4 (2H, m), 857 (1H, dd, J=0.8, 7.9).

### Example 94 CDCl3

0.92 (3H, t, J=7.0), 13-15 (4H, m), 15-1.6 (2H, m), 2.0-2.1 (2H, m), 3.4-3.5 (2H, m), 75-7.6 (3H, m), 7.6-7.7 (1H, m), 7.7-7.8 (1H, m), 8.00 (1H, bd, J=8.2), 8.3-8.4 (2H, m), 857 (1H, dd, J=1.5, 8.2).

### Example 95 CDCl3

0.96 (3H, t, J=73), 1.4-1.6 (4H, m), 2.0-2.1 (2H, m), 3.3-3.4 (2H, m), 7.21 (2H, t, J=8.8), 7.6-7.7 (1H, m), 7.7-7.8 (1H, m), 8.00 (1H, bd, J=8.2), 837 (2H, dd, J=5.6, 8.8), 8.54 (1H, dd, J=1.5, 8.2).

### Example 96 CDCl3

2.4-2.5 (2H, m), 2.60 (2H, t, J=73), 350 (2H, t, J=73), 3.67 (3H, s), 75-7.6 (3H, m), 7.6-7.7 (1H, m), 7.8-7.9 (1H, m), 8.02 (1H, bd, J=8.2), 8.3-8.4 (2H, m), 858 (1H, dd, J=15, 7.9).

### Example 97 CDCl3

2.4-2.5 (2H, m), 2.60 (2H, t, J=7.6), 3.48 (2H, t, J=7.6), 3.67 (3H, s), 7.65 (2H, d, J=8.2), 7.6-7.7 (1H, m), 7.8-7.9 (1H, m), 8.00 (1H, bd, J=8.2), 8.24 (2H, d, J=8.2), 854 (1H, dd, J=1.5, 8.2).

### Example 98 CDCl3

2.4-2.5 (2H, m), 2.60 (2H, t, J=73), 3.48 (2H, t, J=73), 3.67 (3H, s), 7.21 (2H, t, J=9.1), 7.7-7.8 (1H, m), 7.8-7.9 (1H, m), 8.00 (1H, bd, J=8.5), 837 (2H, dd, J=5.6, 9.1), 85-8.6 (1H, m).

### Example 99 CDCl3

2.4-2.5 (2H, m), 2.61 (2H, t, J=73), 3.48 (2H, t, J=7.6), 3.67 (3H, s), 7.49 (2H, d, J=8.2), 7.69 (1H, bt, J=73), 7.8-7.9 (1H, m), 8.01 (1H, bd, J=8.2), 831 (2H, d, J=8.2), 85-8.6 (1H, m).

### Example 100 CDCl3

2.4-2.5 (2H, m), 2.44 (3H, s), 2.60 (2H, t, J=7.6), 3.49 (2H, t, J=73), 3.67 (3H, s), 732 (2H, d, J=8.2)_{,} 7.6-7.7 (1H, m), 7.7-7.8 (1H, m), 8.00 (1H, bd, J=8.2), 8.26 (2H, d, J=8.2), 856 (1H, dd, J=0.9, 7.9).

### Example 101 CDCl3

0.96 (3H, t, J=73), 1.4-1.6 (4H, m), 2.0-2.1 (2H, m), 2.45 (3H, s), 3.4-3.5 (2H, m), 733 (2H, d, J=8.2), 7.6-7.7 (1H, m), 7.7-7.8 (1H, m), 8.00 (1H, bd, J=8.2), 8.27 (2H, d, J=8.2), 8.56 (1H, dd, J=1.5, 7.9).

### Example 102 CDCl3

0.96 (3H, t, J=73), 1.4-1.6 (4H, m), 2.0-2.1 (2H, m), 3.3-3.4 (2H, m), 7.65 (2H, d, J=8.5), 7.6-7.7 (1H, m), 7.8-7.9 (1H, m), 8.01 (1H, bd, J=85), 8.26 (2H, d, J=8.5), 854 (1H, dd, J=1.5, 8.2).

### Example 103 CDCl3

0.96 (3H, t, J=73), 1.4-1.6 (4H, m), 2.0-2.1 (2H, m), 3.3-3.4 (2H, m), 7.49 (2H, d, J=8.2), 7.6-7.7 (1H, m), 7.8-7.9 (1H, m), 8.01 (1H, bd, J=8.5), 832 (2H, d, J=8.2), 854 (1H, dd, J=1.5, 7.9).

### Example 104 CDCl3

23-25 (2H, m), 2.60 (2H, t, J=73), 3.46 (2H, t, J=73), 3.67 (3H, s), 7.49 (2H, d, J=8.5), 7.74 (1H, dd, J=23, 8.8), 7.93 (1H, d, J=8.8), 8.29 (2H, d, J=85), 852 (1H, d, J=2.3).

### Example 105 CDCl3

0.96 (3H, t, J=7.0), 1.4-1.6 (4H, m), 2.0-2.1 (2H, m), 33-3.4 (2H, m), 3.90 (3H, s), 7.04 (2H, d, J=9.1), 7.6-7.7 (1H, m), 7.7-7.8 (1H, m), 7.99 (1H, bd, J=8.5), 832 (2H, d, J=9.1), 8.55 (1H, dd, J=0.9, 7.9).

### Example 106 CDCl3

0.96 (3H, t, J=73), 1.47 (3H, t, J=7.0), 1.4-1.6 (4H, m), 2.0-2.1 (2H, m), 33-3.4 (2H, m), 4.12 (2H, q, J=7.0), 7.02 (2H, d, J=8.5), 7.6-7.7 (1H, m), 7.7-7.8 (1H, m), 7.99 (1H, bd, J=8.2), 830 (2H, d, J=85), 854 (1H, bd, J=7.9).

### Example 107 CDCl3

0.95 (3H, t, J=7.1), 0.96 (3H, t, J=7.1), 1.4-1.6 (8H, m), 1.7-1.8 (2H, m), 2.0-2.1 (2H, m), 25-2.6 (2H, m), 3.4-3.5 (2H, m), 7.26 (2H, d, J=8.7), 7.6-7.7 (1H, m), 7.8-7.9 (1H, m), 8.01 (1H, bd, J=7.9), 8.41 (2H, d, J=8.7), 856 (1H, dd, J=0.8,7.9).

### Example 108 DMSO-d6

0.99 (3H, t, J=73), 1.4-1.6 (2H, m), 1.9-2.0 (2H, m), 33-3.4 (2H, m), 4.10 (3H, s), 7.40 (2H, t, J=8.8), 8.29 (2H, dd, J=5.6, 8.8), 8.45 (1H, s).

### Example 109 DMSO-d6

0.98 (3H, t, J=73), 1.4-1.5 (2H, m), 1.9-2.0 (2H, m), 3.2-33 (2H, m), 4.17 (3H, s), 739 (2H, t, J=8.8), 8.25 (2H, dd, J=5.6, 8.8), 8.82 (1H, s).

### Example 110 DMSO-d6

0.99 (3H, t, J=73), 15-1.6 (2H, m), 1.9-2.0 (2H, m), 33-3.4 (2H, m), 4.11 (3H, s), 75-7.6 (3H, m), 8.2-83 (2H, m), 8.47 (1H, s).

### Example 111 DMSO-d6

0.98 (3H, t, J=73), 1.4-1.5 (2H, m), 1.9-2.0 (2H, m), 3.3-3.4(2H, m), 4.17 (3H, s), 75-7.6 (3H, m), 8.2-83 (2H, m), 8.84 (1H, s).

### Example 112 DMSO-d6

0.86 (3H, t, J=7.6), 0.99 (3H, t, J=7.3), 1.4-1.5 (2H, m), 1.9-2.0 (4H, m), 3.3-3.4 (2H, m), 4.45 (2H, t, J=7.0), 75-7.6 (3H, m), 8.2-83 (2H, m), 8.48 (1H, s).

### Example 113 DMSO-d6

0.88 (3H, t, J=7.3), 0.99 (3H, t, J=7.3), 1.4-1.5 (2H, m), 1.9-2.0 (4H, m), 3.3-3.4 (2H, m), 439 (2H, t, J=7.0), 75-7.6 (3H, m), 8.2-83 (2H, m), 8.90 (1H, s).

### Example 114 DMSO-d6

0.99 (3H, t, J=7.6), 1.49 (3H, t, J=73), 1.4-1.5 (2H, m), 1.9-2.0 (2H, m), 33-3.4 (2H, m), 452 (2H, q, J=73), 75-7.6 (3H, m), 8.2-83 (2H, m), 8.48 (1H, s).

### Example 115 DMSO-d6

0.98 (3H, t, J=73), 1.4-1.5 (2H, m), 153 (3H, t, J=73), 1.9-2.0 (2H, m), 3.3-3.4 (2H, m), 4.46 (2H, q, J=73), 75-7.6 (3H, m), 8.2-83 (2H, m), 8.91 (1H, s).

### Example 116 DMSO-d6

0.99 (3H,t,J=73), 1.4-1.5 (2H,m), 1.9-2.0 (2H,m), 33-3.4 (2H,m), 5.71 (2H,s), 7.2-7.4 (5H, m), 75-7.6 (3H, m), 8.2-83 (2H, m), 8.51 (1H, s).

### Example 117 DMSO-d6

0.97 (3H, t, J=73), 1.4-1.5 (2H, m), 1.8-1.9 (2H, m), 3.2-33 (2H, m), 5.65 (2H, s), 7.3-7.4 (5H, m), 75-7.6 (3H, m), 8.2-83 (2H, m), 9.05 (1H, s).

### Example 118 DMSO-d6

0.99 (3H, t, J=7.6), 1.4-1.6 (2H, m), 1.9-2.0 (2H, m), 3.3-3.4 (2H, m), 4.08 (3H, s), 7.60 (2H, d, J=8.5), 8.20 (2H, d, J=85), 8.42 (1H, s).

### Example 119 DMSO-d6

0.98 (3H, t, J=7.3), 1.4-15 (2H, m), 1.9-2.0 (2H, m), 3.2-3.3 (2H, m), 4.17 (3H, s), 7.62 (2H, d, J=85), 8.21 (2H, d, J=85), 8.83 (1H, s).

### Example 120 DMSO-d6

0.99 (3H, t, J=7.3), 1.4-1.6 (2H, m), 1.9-2.0 (2H, m), 2.40 (3H, s), 3.3-3.4 (2H, m), 4.10 (3H, s), 738 (2H, d, J=7.9), 8.15 (2H, d, J=7.9), 8.45 (1H, s).

### Example 121 DMSO-d6

0.97 (3H, t, J=73), 1.4-1.5 (2H, m), 1.9-2.0 (2H, m), 239 (3H, s), 3.2-33 (2H, m), 4.16 (3H, s), 736 (2H, d, J=7.9), 8.11 (2H, d, J=7.9), 8.81 (1H, s).

### Example 122 DMSO-d6

0.86 (3H, t, J=75), 0.99 (3H, t, J=75), 1.4-1.6 (2H, m), 1.9-2.0 (4H, m), 2.40 (3H, s), 33-3.4 (2H, m), 4.45 (2H, t, J=7.1), 738 (2H, d, J=7.9), 8.15 (2H, d, J=7.9), 8.46 (1H, s).

### Example 123 DMSO-d6

0.88 (3H, t, J=75), 0.98 (3H, t, J=75), 1.4-1.5 (2H, m), 1.8-2.0 (4H, m), 239 (3H, s), 3.2-33 (2H, m), 438 (2H, t, J=7.1), 736 (2H, d, J=7.9), 8.11 (2H, d, J=7.9), 8.87 (1H, s).

### Example 124 DMSO-d6

0.86 (3H, t, J=75), 0.99 (3H, t, J=75), 1.4-1.6 (2H, m), 1.9-2.0 (4H, m), 3.3-3.4 (2H, m), 4.45 (2H, t, J=7.1), 7.40 (2H, t, J=8.7), 830 (2H, dd, J=5.4, 8.7), 8.47 (1H, s).

### Example 125 DMSO-d6

0.88 (3H, t, J=75), 0.98 (3H, t, J=75), 1.4-15 (2H, m), 1.9-2.0 (4H, m), 3.2-33 (2H, m), 439 (2H, t, J=7.1), 739 (2H, t, J=8.7), 8.26 (2H, dd, J=5.4, 8.7), 8.89 (1H, s).

### Example 126 DMSO-d6

0.86 (3H, t, J=75), 0.98 (3H, t, J=75), 1.4-1.6 (2H, m), 1.9-2.0 (4H, m), 3.3-3.4 (2H, m), 4.45 (2H, t, J=7.1), 7.61 (2H, d, J=8.7), 8.25 (2H, d, J=8.7), 8.47 (1H, s).

### Example 127 DMSO-d6

0.88 (3H, t, J=75), 0.98 (3H, t, J=75), 1.4-1.5 (2H, m), 1.9-2.0 (4H, m), 3.2-33 (2H, m), 439 (2H, t, J=7.1), 7.63 (2H, d, J=8.7), 8.22 (2H, d, J=8.7), 8.90 (1H, s).

### Example 128 DMSO-d6

0.97 (3H, t, J=73), 1.22 (3H, t, J=7.0), 1.4-1.5 (2H, m), 1.9-2.0 (2H, m), 3.3-3.4 (2H, m), 4.19 (2H, q, J=7.0), 5.41 (2H, s), 7.5-7.6 (3H, m), 8.2-83 (2H, m), 8.56 (1H, s).

### Example 129 DMSO-d6

0.99 (3H, t, J=73), 1.25 (3H, t, J=7.0), 1.4-1.5 (2H, m), 1.9-2.0 (2H, m), 3.3-3.4 (2H, m), 4.21 (2H, q, J=7.0), 5.43 (2H, s), 75-7.6 (3H, m), 8.2-83 (2H, m), 8.89 (1H, s).

### Example 130 DMSO-d6

0.97 (3H, t, J=73), 1.22 (3H, t, J=7.0), 1.4-1.5 (2H, m), 1.9-2.0 (2H, m), 3.3-3.4 (2H, m), 4.19 (2H, q, J=7.0), 5.41 (2H, s), 7.41 (2H, t, J=8.8), 830 (2H, dd, J=5.6, 8.8), 855 (1H, s).

### Example 131 DMSO-d6

0.98 (3H, t, J=73), 1.24 (3H, t, J=7.0), 1.4-1.5 (2H, m), 1.9-2.0 (2H, m), 3.2-33 (2H, m), 4.21 (2H, q, J=7.0), 5.43 (2H, s), 7.40 (2H, t, J=8.8), 8.27 (2H, dd, J=5.6, 8.8), 8.88 (1H, s).

### Example 132 DMSO-d6

0.99 (3H, t, J=7.6), 1.4-1.5 (2H, m), 1.9-2.0 (2H, m), 3.3-3.4 (2H, m), 3.91 (2H, bq, J=5.6), 4.52 (2H, bt, J=5.6), 4.93 (1H, bt, J=5.6), 75-7.6 (3H, m), 8.2-83 (2H, m), 8.46 (1H, s).

### Example 133 DMSO-d6

0.98 (3H, t, J=73), 1.4-15 (2H, m), 1.9-2.0 (2H, m), 3.3-3.4 (2H, m), 3.90 (2H, bq, J=53), 4.47 (2H, bt, J=53), 5.03 (1H, bt, J=5.3), 75-7.6 (3H, m), 8.2-83 (2H, m), 8.83 (1H, s).

### Example 134 DMSO-d6

0.99 (3H, t, J=73), 1.4-1.5 (2H, m), 1.9-2.0 (2H, m), 33-3.4 (2H, m), 3.91 (2H, bq, J=5.6), 451 (2H, bt, J=5.6), 4.92 (1H, bt, J=5.6), 738 (2H, t, J=8.8), 8.26 (2H, dd, J=5.6, 8.8), 8.45 (1H,s).

### Example 135 DMSO-d6

0.98 (3H, t, J=7.3), 1.4-1.5 (2H, m), 1.8-2.0 (2H, m), 3.2-33 (2H, m), 3.90 (2H, bq, J=5.3), 4.46 (2H, bt, J=53), 5.03 (1H, bt, J=5.3), 739 (2H, t, J=8.8), 8.25 (2H, dd, J=5.6, 8.8), 8.82 (1H, s).

### Example 136 DMSO-d6

0.99 (3H, t, J=73), 1.4-15 (2H, m), 1.9-2.0 (2H, m), 2.40 (3H, s), 33-3.4 (2H, m), 3.91 (2H, bq, J=5.9), 452 (2H, bt, J=5.9), 4.90 (1H, bt, J=5.9), 737 (2H, d, J=8.2), 8.14 (2H, d, J=8.2), 8.46 (1H, s).

### Example 137 DMSO-d6

0.98 (3H, t, J=73), 1.4-1.5 (2H, m), 1.9-2.0 (2H, m), 239 (3H, s), 3.2-33 (2H, m), 3.90 (2H, bt, J=5.6), 4.46 (2H, bt, J=5.6), 736 (2H, d, J=8.2), 8.11 (2H, d, J=8.2), 8.81 (1H, s).

### Example 138 DMSO-d6

0.99 (3H, t, J=75), 1.4-1.6 (2H, m), 1.9-2.0 (2H, m), 3.3-3.4 (2H, m), 3.91 (2H, bq, J=5.8), 453 (2H, bt, J=5.8), 4.90 (1H, bt, J=5.8), 7.63 (2H, d, J=83), 8.25 (2H, d, J=83), 8.47 (1H, s).

### Example 139 DMSO-d6

0.98 (3H, t, J=7.5), 1.4-1.5 (2H, m), 1.8-2.0 (2H, m), 3.2-3.3 (2H, m), 3.9-4.0 (2H, m), 4.46 (2H, bt, J=5.4), 5.0-5.1 (1H, m), 7.63 (2H, d, J=8.7), 8.23 (2H, d, J=8.7), 8.83 (1H, s).

### Example 40 DMSO-d6

0.99 (3H, t, J=75), 1.4-15 (2H, m), 1.9-2.0 (2H, m), 3.3-3.4 (2H, m), 3.85 (3H, s), 3.91 (2H, bq, J=5.8), 452 (2H, bt, J=5.8), 4.90 (1H, bt, J=5.8), 7.11 (2H, d, J=8.7), 8.18 (2H, d, J=8.7), 8.45 (1H, s).

### Example 141 DMSO-d6

0.98 (3H, t, J=75), 1.4-1.5 (2H, m), 1.8-2.0 (2H, m), 3.2-33 (2H, m), 3.85 (3H, s), 3.8-3.9 (2H, m), 4.46 (2H, bt, J=5.4), 5.0-5.1 (1H, m), 7.10 (2H, d, J=9.1), 8.15 (2H, d, J=9.1), 8.80 (1H, s).

### Example 142 DMSO-d6

0.98 (3H, t, J=75), 1.4-15 (2H, m), 153 (3H, t, J=75), 1.9-2.0 (2H, m), 3.2-33 (2H, m), 3.85 (3H, s), 4.45 (2H, q, J=75), 7.11 (2H, d, J=8.7), 8.16 (2H, d, J=8.7), 8.88 (1H, s).

### Example 143 DMSO-d6

0.98 (3H, t, J=75), 1.4-15 (2H, m), 1.53 (3H, t, J=75), 1.9-2.0 (2H, m), 2.40 (3H, s), 3.2-33 (2H, m), 4.46 (2H, q, J=75), 737 (2H, d, J=7.9), 8.12 (2H, d, J=7.9), 8.89 (1H, s).

### Example 144 DMSO-d6

0.98 (3H, t, J=75), 1.4-1.5 (2H, m), 153 (3H, t, J=75), 1.9-2.0 (2H, m), 3.2-33 (2H, m), 4.46 (2H, q, J=75), 7.40 (2H, t, J=8.7), 8.26 (2H, dd, J=5.4, 8.7), 8.90 (1H, s).

### Example 145 DMSO-d6

0.98 (3H, t, J=75), 1.4-1.5 (2H, m), 153 (3H, t, J=75), 1.9-2.0 (2H, m), 3.2-33 (2H, m), 4.46 (2H, q, J=75), 7.63 (2H, d, J=8.7), 8.23 (2H, d, J=8.7), 8.90 (1H, s).

### Example 146 DMSO-d6

0.87 (3H, t, J=75), 0.98 (3H, t, J=75), 1.4-1.5 (2H, m), 1.8-2.0 (4H, m), 3.2-33 (2H, m), 3.85 (3H, s), 438 (2H, t, J=7.0), 7.11 (2H, d, J=8.7), 8.16 (2H, d, J=8.7), 8.88 (1H, s).

### Example 147 DMSO-d6

2.1-23 (2H, m), 2.48 (2H, t, J=75), 3.40 (2H, t, J=75), 75-7.6 (3H, m), 8.2-8.3 (2H, m), 8.56 (1H, bs).

### Example 148 DMSO-d6

2.1-2.2 (2H, m), 2.47 (2H, t, J=7.5), 338 (2H, t, J=75), 3.85 (3H, s), 7.11 (2H, d, J=8.7), 8.19 (2H, d, J=8.7), 853 (1H, bs).

### Example 149 DMSO-d6

2.1-2.2 (2H, m), 2.47 (2H, t, J=7.5), 3.39 (2H, t, J=75), 7.63 (2H, d, J=83), 8.26 (2H, d, J=8.3), 8.56 (1H, bs).

### Example 150 DMSO-d6

2.1-2.2 (2H, m), 2.40 (3H, s), 2.47 (2H, t, J=75), 339 (2H, t, J=7.5), 737 (2H, d, J=7.9), 8.15 (2H, d, J=7.9), 854 (1H, bs).

### Example 151 DMSO-d6

0.98 (3H, t, J=75), 1.4-15 (2H, m), 1.9-2.0 (2H, m), 3.3-3.4 (2H, m), 5.29 (2H, s), 75-7.6 (3H, m), 8.2-83 (2H, m), 852 (1H, s).

### Example 152 DMSO-d6

0.97 (3H, t, J=75), 1.4-1.5 (2H, m), 1.9-2.0 (2H, m), 3.2-33 (2H, m), 5.29 (2H, s), 75-7.6 (3H, m), 8.2-83 (2H, m), 8.84 (1H, s).

### Example 153 DMSO-d6

2.2-23 (2H, m), 250 (2H, t, J=75), 3.40 (2H, t, J=75), 75-7.6 (3H, m), 7.77 (1H, bt, J=7.9), 7.91 (1H, bt, J=7.9), 8.01 (1H, bd, J=7.9), 8.2-83 (2H, m), 8.46 (1H, bd, J=7.9).

### Example 154 DMSO-d6

2.2-23 (2H, m), 2.49 (2H, t, J=7.5), 339 (2H, t, J=75), 7.7-7.8 (1H, m), 7.77 (2H, d, J=8.3), 7.9-8.0 (1H, m), 8.01 (1H, bd, J=83), 8.20 (2H, d, J=8.3), 8.45 (1H, bd, J=7.9).

### Example 155 DMSO-d6

2.2-2.3 (2H, m), 2.49 (2H, t, J=7.5), 339 (2H, t, J=7.5), 7.41 (2H, t, J=8.7), 7.78 (1H, bt, J=7.5), 7.92 (1H, bt, J=7.5), 8.01 (1H, bd, J=75), 832 (2H, dd, J=5.8, 8.7), 8.46 (1H, bd, J=7.5).

### Example 156 DMSO-d6

2.2-2.3 (2H,m), 2.49 (2H,t,J=75), 338 (2H,t,J=75), 7.63 (2H,d,J=8.3), 7.77 (1H,bt, J=7.9), 7.91 (1H, bt, J=7.9), 8.00 (1H, bd, J=7.9), 8.26 (2H, d, J=83), 8.44 (1H, bd, J=7.9).

### Example 157 DMSO-d6

0.91 (3H, t, J=7.1), 1.3-1.5 (4H, m), 1.9-2.0 (2H, m), 3.3-3.4 (2H, m), 6.94 (2H, d, J=8.7), 7.75 (1H, bt, J=7.9), 7.8-7.9 (1H, m), 7.99 (1H, bd, J=83), 8.11 (2H, d, J=8.7), 8.44 (1H, dd, J=0.8, 7.9).

### Experiment Example

### Adenosine A3 Receptor Binding Capacity Test of Triazolopurine Derivative (1)

According to the method described in Molecular Pharmacology, 45, 978 (1994), an adenosine A3 receptor binding capacity test was performed.

A cell membrane of human renal endothelial cells HEK-293 transformed with plasmid coding an adenosine A3 receptor was isolated in a Tris-hydrochloric acid buffer (pH 7.7) in accordance with a conventional method, and then the cell membrane was treated with N⁶-(4-aminobenzyl)-9-[5-(methylcarbonyl)- β-D-ribofuranosyl]adenine (AB-MECA) labelled with ¹²⁵I to prepare a cell membrane bound with the compound.

Then, this cell membrane and a test compound were incubated and the amount of [¹²⁵I] AB-MECA liberated was measured. The concentration of the test compound when 50% of [¹²⁵I] AB-MECA is liberated, IC₅₀, was determined from the measured value of the test compound at each concentration.

The adenosine A2 receptor binding capacity of the test compound were measured according to the method described in Archives of Pharmacology, 336, 204 (1987) and The Journal of Pharmacology and Experimental Therapeutics, 251 (3), 888 (1989) and then evaluated as IC₅₀. The measurement results are shown in the following tables.

**Table 1**

| Example No. | Receptor Binding Capacity (IC₅₀) (nM) | |
|---|---|---|
| | Adenosine A2 | Adenosine A3 |
| 4 | 189 | <10 |
| 5 | >10000 | <10 |
| 6 | >10000 | <10 |
| 7 | >10000 | 19 |
| 8 | >10000 | <10 |
| 9 | >10000 | 154 |
| 10 | >10000 | 132 |
| 13 | 2723 | <10 |
| 22 | > 10000 | 255 |
| 23 | > 10000 | 28 |
| 26 | > 10000 | 26 |
| 27 | > 10000 | <10 |
| 28 | > 10000 | 34 |
| 31 | > 10000 | 144 |
| 32 | 5311 | 10 |
| 34 | > 10000 | 155 |
| 35 | > 10000 | 35 |
| 37 | 1445 | 199 |
| 45 | > 10000 | 45 |
| 46 | > 10000 | 48 |
| 47 | > 10000 | 93 |
| 50 | > 10000 | 109 |
| 54 | 2495 | <10 |
| 55 | 2569 | <10 |
| 61 | > 10000 | 12 |
| 62 | > 10000 | 42 |
| 73 | > 10000 | 71 |
| 74 | > 10000 | 63 |
| 75 | > 10000 | 28 |
| 76 | > 10000 | 22 |
| 77 | > 10000 | 23 |
| 78 | > 10000 | 33 |
| 83 | > 10000 | 30 |
| 84 | > 10000 | 34 |
| 87 | 2859 | <10 |
| 93 | > 10000 | 37 |
| 113 | 4526 | 12 |
| 115 | 1563 | <10 |
| 133 | 4160 | 24 |
| 152 | > 10000 | 32 |

### Preparation Example 1

### (Preparation of tablets)

Two-thousands tablets, each of which contains 300 mg of the compound (5-n-butyl-2-phenylpyrazolo[4,3-e]-1,2,4-triazolo[1,5-c]pyrimidine) obtained in Example 4 as an active ingredient, were prepared according to the following formulation.

| | |
|---|---|
| Compound obtained in Example 4 | 600 g |
| Lactose (Japanese Pharmacopoeia) | 67 g |
| Cornstarch (Japanese Pharmacopoeia) | 33 g |
| Calcium carboxymethylcellulose (Japanese Pharmacopoeia) | 25 g |
| Methylcellulose (Japanese Pharmacopoeia) | 12g |
| Magnesium stearate (Japanese Pharmacopoeia) | 3g |

According to the formulation described above, desired tablets were obtained by sufficiently mixing the compound obtained in Example 4, lactose, cornstarch and calcium carboxymethylcellulose, granulating the resulting mixture using an aqueous methylcellulose, passing the granules through a #24 mesh sieve, admixing the granules with magnesium stearate and compressing the admixture into tablets.

### Preparation Example 2

### (Preparation of capsules)

Two-thousands hard gelatin capsules, each of which contains 200 mg of the compound (5-n-butyl-2-(3,4,5-trimethoxyphenyl)-1,2,4-triazolo[1,5-c] quinazoline) obtained in Example 37 as an active ingredient, were prepared according to the following formulation.

| | |
|---|---|
| Compound obtained in Example 37 | 400 g |
| Crystalline cellulose (Japanese Pharmacopoeia) | 60 g |
| Cornstarch (Japanese Pharmacopoeia) | 34 g |
| Talc (Japanese Pharmacopoeia) | 4 g |
| Magnesium stearate (Japanese Pharmacopoeia) | 2 g |

According to the formulation described above, desired capsules were obtained by pulverizing the respective ingredients to form powders, mixing the powders to obtain an uniform mixture and filling a gelatin capsule for oral administration having a desired size with the mixture.

### Preparation Example 3

### (Preparation of opthalmic solutions)

Opthalmic solutions were prepared by dissolving 100 g of the compound obtained in Example 152,10 g of benzalkonium chloride, 560 g of sodium bihydrogenphosphate and 800 g of potassium bihydrogenphosphate in water for injection to make 100 l and filling a container with the solution.

## Claims

1. Triazoloquinazoline and pyrazolotriazolopyrimidine derivatives represented by the general formula (1): 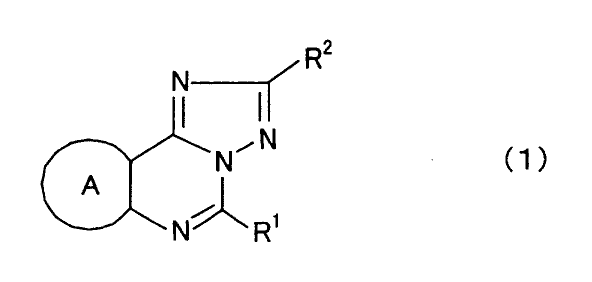 wherein R¹ represents a lower alkyl group, a phenyl group, a lower alkoxycarbonyl lower alkyl group, or a carboxy lower alkyl group; R² represents a pyridyl group, a furyl group, a thienyl group, or a phenyl group which optionally has 1 to 3 groups selected from lower alkyl group, halogen atom, phenyl group, halogen-substituted lower alkyl group, hydroxy group, lower alkoxy group, N,N-di lower alkylamino group, lower alkylthio group, lower alkanoyloxy group and nitro group as a substituent; A represents a pyrazole ring which is optionally substituted with a group selected from lower alkyl group, phenyl lower alkyl group, lower alkoxycarbonyl lower alkyl group, carboxy lower alkyl group and hydroxy lower alkyl group as a substituent, or a benzene ring which is optionally substituted with 1 to 2 groups selected from halogen atom, lower alkyl group, nitro group and lower alkoxy group as a substituent; with the exception that A is a benzene ring, R² is a pyridyl group or a phenyl group, and R¹ is a methyl group, an ethyl group or a phenyl group.

2. Triazoloquinazoline and pyrazolotriazolopyrimidine derivatives according to claim 1, wherein the pyrazolotriazolopyrimidine derivative is represented by the following general formula (1-1): 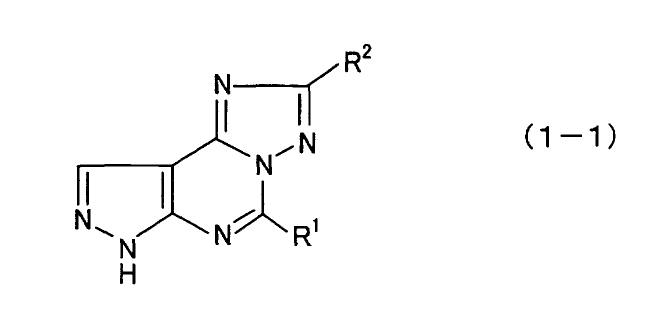 wherein R¹ and R² are as defined above.

3. Triazoloquinazoline and pyrazolotriazolopyrimidine derivatives according to claim 1 or 2, wherein R² is a phenyl group which optionally have one group selected from lower alkyl group, halogen atom, halogen-substituted lower alkyl group and hydroxy group as a substituent, or phenyl group which has 1 to 3 lower alkoxy groups.

4. Triazoloquinazoline and pyrazolotriazolopyrimidine derivatives according to claim 3, wherein A is a pyrazole ring, or a benzene ring which is optionally substituted with one halogen atom as a substituent.

5. Triazoloquinazoline and pyrazolotriazolopyrimidine derivatives according to claim 4, wherein R¹ is an n-butyl group and R² is a phenyl group which optionally has one group selected from lower alkoxy group, lower alkyl group, halogen atom and hydroxy group as a substituent.

6. Triazoloquinazoline and pyrazolotriazolopyrimidine derivatives according to claim 5, which are selected from compound in which A is a pyrazole ring and R² is a phenyl group having any one of lower alkoxy group, lower alkyl group or halogen atom as a substituent, compound in which A is a benzene ring and R² is a phenyl group having one group selected from lower alkoxy group, halogen atom and hydroxy group as a substituent, and compound in which A is a benzene ring substituted with one halogen atom and R² is a phenyl group.

7. Triazoloquinazoline and pyrazolotriazolopyrimidine derivatives according to claim 6, which are selected from 5-n-butyl-2-(4-chlorophenyl)pyrazolo[4,3-e]-1,2,4-triazolo[1,5-c]pyrimidine, 5-n-butyl-2-(4-chlorophenyl)-1,2,4-triazolo[1,5-c]quinazoline, 5-n-butyl-2-(4-ethoxyphenyl)-1,2,4-triazolo[1,5-c]quinazoline and 5-n-butyl-9-chloro-2-phenyl-1,2,4-triazolo[1,5-c]quinazoline.

8. Triazoloquinazoline and pyrazolotriazolopyrimidine derivatives according to claim 5, wherein R² is a phenyl group which has a lower alkyl group or a halogen atom at the 4-position.

9. Triazoloquinazoline and pyrazolotriazolopyrimidine derivatives according to claim 8, which are selected from 5-n-butyl-2-(4-chlorophenyl)pyrazolo[4,3-e]-1,2,4-triazolo[1,5-c]pyrimidine, 5-n-butyl-2-(4-fluorophenyl)pyrazolo[4,3-e]-1,2,4-triazolo[1,5-c]pyrimidine, 5-n-butyl-2-(4-methylphenyl)pyrazolo[4,3-e]-1,2,4-triazolo[1,5-c]pyrimidine, 5-n-butyl-2-(4-chlorophenyl)-1,2,4-triazolo[1,5-c]quinazoline, 5-n-butyl-2-(4-bromophenyl)-1,2,4-triazolo[1,5-c]quinazoline and 5-n-butyl-2-(4-chlorophenyl)-9-chloro-1 ,2,4-triazolo[1,5-c]quinazoline.

10. Triazoloquinazoline and pyrazolotriazolopyrimidine derivatives according to claim 9, which are selected from 5-n-butyl-2-(4-methylphenyl)pyrazolo[4,3-e]-1,2,4-triazolo[1,5-c]pyrimidine and 5-n-butyl-2-(4-bromophenyl)-1,2,4-triazolo[1,5-c]quinazoline.

11. A pharmaceutical composition comprising a compound selected from the triazoloquinazoline and pyrazolotriazolopyrimidine derivatives of any one of claims 1 to 10, and a pharmaceutically acceptable carrier.

12. An adenosine A3 receptor affinitive agent comprising a compound selected from the triazoloquinazoline and pyrazolotriazolopyrimidine derivatives of any one of claims 1 to 10 as an active ingredient.

13. An intraocular-pressure reducing agent comprising a compound selected from the triazoloquinazoline and pyrazolotriazolopyrimidine derivatives of any one of claims 1 to 10 as an active ingredient.

14. A pharmaceutical preparation for prevention or treatment of glaucoma, comprising a compound selected from the triazoloquinazoline and pyrazolotriazolopyrimidine derivatives of any one of claims 1 to 10 as an active ingredient.

15. An intraocular-pressure reducing method, which comprises administering an effective amount of a compound selected from the triazoloquinazoline and pyrazolotriazolopyrimidine derivatives of claims 1 to 10 to a patient having an enhanced intraocular pressure.

16. Use of a compound selected from the triazoloquinazoline and pyrazolotriazolopyrimidine derivatives of claims 1 to 10 for reduction of an intraocular pressure of a patient having an enhanced intraocular pressure.

17. Use of a compound selected from the triazoloquinazoline and pyrazolotriazolopyrimidine derivatives of claims 1 to 10 for production of a preventive or remedy for glaucoma.
